# EUROPÄISCHE PATENTSCHRIFT

(11) **EP 2 753 615 B1**
(45) Veröffentlichungstag und Bekanntmachung des Hinweises auf die Patenterteilung: **19.07.2017**
(21) Anmeldenummer: 12747896.4
(22) Anmeldetag: 13.08.2012
(51) Int. Cl.: C07D 401/12, C07D 401/14, C07D 405/14, C07D 413/14, C07D 417/14, C07D 471/04, C07D 491/10, A61K 31/506, A61K 31/4433

(54) **BENZONITRILDERIVATE ALS KINASEHEMMER**
BENZONITRILE DERIVATIVES AS KINASE INHIBITORS
DÉRIVÉS DE BENZONITRILE EN TANT QU'INHIBITEURS DE KINASES

(30) Priorität: 09.09.2011 DE 102011112978
(43) Veröffentlichungstag der Anmeldung: 16.07.2014
(73) Patentinhaber: Merck Patent GmbH, 64293 Darmstadt (DE)
(72) Erfinder: HOELZEMANN, Guenter, 64342 Seeheim-Jugenheim (DE); DORSCH, Dieter, 64372 Ober-Ramstadt (DE); EGGENWEILER, Hans-Michael, 64291 Darmstadt (DE)
(86) Internationale Anmeldenummer: PCT/EP2012/003449
(87) Internationale Veröffentlichungsnummer: WO 2013/034238

(56) Entgegenhaltungen:
- WO-A1-2005/012262
- WO-A1-2008/065155
- WO-A1-2008/109943
- WO-A1-2009/032861
- WO-A1-2010/151747
- WO-A1-2011/046970
- WO-A1-2012/010826
- WO-A1-2012/062704
- WO-A2-2004/016597

## Beschreibung

### Hintergrund der Erfindung

Es war die Aufgabe der Erfindung, neue Verbindungen mit wertvollen Eigenschaften aufzufinden, insbesondere solche, die für die Herstellung von Arzneimitteln verwendet werden können.
Die vorliegende Erfindung betrifft Benzonitrilverbindungen, die in der Lage sind, eine oder mehrere Kinasen zu hemmen. Die Verbindungen finden Verwendung in der Behandlung einer Vielzahl von Störungen, darunter Krebs, septischer Schock, primäres Offenwinkelglaukom (Primary open Angle Glaucoma - POAG), Hyperplasie, rheumatoide Arthritis, Psoriasis, Atherosklerose, Retinopathie, Osteoarthritis, Endometriose, chronische Entzündung und/oder neurodegenerative Erkrankungen wie Morbus Alzheimer.

Die vorliegende Erfindung betrifft Verbindungen und die Verwendung von Verbindungen, bei denen die Hemmung, Regulierung und/oder Modulation der Signaltransduktion von Rezeptorkinasen eine Rolle spielt, ferner pharmazeutische Zusammensetzungen, die diese Verbindungen enthalten, sowie die Verbindungen zur Verwendung zur Behandlung kinasebedingter Krankheiten.
Da Proteinkinasen nahezu jeden Zellprozeß, darunter Metabolismus, Zellproliferation, Zelldifferenzierung und Zellüberleben, regulieren, stellen sie attraktive Ziele für therapeutische Eingriffe bei verschiedenen Krankheitszuständen dar. Beispielsweise sind Zellzyklussteuerung und Angiogenese, in denen Proteinkinasen eine Schlüsselrolle spielen, Zellvorgänge, die mit zahlreichen Krankheitszuständen wie Krebs, Entzündungskrankheiten, abnormale Angiogenese und damit in Zusammenhang stehende Krankheiten, Atherosklerose, Makula-Degeneration, Diabetes, Fettsucht und Schmerz einhergehen, ohne hierauf beschränkt zu sein.

Die vorliegende Erfindung betrifft insbesondere Verbindungen und die Verwendung von Verbindungen, bei denen die Hemmung, Regulierung und/oder Modulation der Signaltransduktion von TBK1 und IKKε eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

IKKε und TBK1 sind Serin/Threonin Kinasen die hohe Homologien untereinander sowie zu anderen IkB-Kinasen aufweisen. Beide Kinasen spielen eine integrale Rolle für das angeborene, immanente Immunsystem. Doppelsträngige RNA-Viren werden durch die Toll-like Rezeptoren 3 und 4, sowie die RNA-Helicasen RIG-I und MDA-5 erkannt und führen zu einer Aktivierung der TRIF-TBK1/IKKε-IRF3 Signalkaskade, was zu einer Typ I Interferon-Antwort führt.

Boehm und Kollegen beschrieben 2007 IKKε als ein neuartiges Brustkrebsonkogen [J.S. Boehm et al., Cell 129, 1065-1079, 2007]. 354 Kinasen wurden auf Ihre Fähigkeit hin untersucht gemeinschaftlich mit einer aktivierten Form der MAPK Kinase Mek, den Ras-transformierenden Phänotyp zu rekapitulieren. IKKε wurde hierbei als ein kooperatives Onkogen identifiziert. Darüber hinaus konnten die Autoren zeigen, dass IKKε in zahlreichen Brustkrebszelllinien und Tumorproben amplifiziert und überexprimiert vorliegt. Die Verminderung der Genexpression mittels RNA interference in Brustkrebszellen induziert Apoptosis und beeinträchtigt deren Proliferation. Eddy und Kollegen kamen 2005 zu ähnlichen Befunden, was die Bedeutung von IKKε in Brustkrebserkrankungen unterstreicht [S.F.Eddy et al., Cancer Res. 2005; 65 (24), 11375-11383].

Über einen protumorigenen Effekt von TBK1 wurde erstmals 2006 berichtet. Korherr und Kollegen identifizierten in einem Screening einer 251000 cDNA umfassenden Genbibliothek mit TRIF, TBK1 und IRF3 gleich drei Gene, die typischerweise in der angeborenen Immunabwehr involviert sind, als proangiogene Faktoren [C.Korherr et al., PNAS, 103, 4240-4245, 2006]. Chien und Kollegen publizierten 2006 [Y.Chien et al., Cell 127, 157-170, 2006], dass TBK1-/- Zellen nur bedingt mit oncogenem Ras transformierbar sind, was eine Involvierung von TBK1 bei der Rasvermittelten Transformation nahelegt. Desweiteren konnten Sie zeigen, dass ein RNAi vermittelter knock down von TBK1 Apoptose in MCF-7 und Panc-1 Zellen auslöst. Kürzlich publizierten Barbie und Kollegen, dass TBK1 in zahlreichen Krebszellinien mit mutierten K-Ras von essentieller Bedeutung ist, was nahelegt, dass eine TBK1 Intervention in entsprechenden Tumoren von therapeutischer Bedeutung sein könnte [D.A.Barbie et al., Nature Letters 1-5, 2009].

Durch Proteinkinasen hervorgerufene Erkrankungen sind durch eine anomale Aktivität oder Hyperaktivität solcher Proteinkinasen gekenn zeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

IKKε und TBK1 sind hochgradig homologe Ser/Thr-Kinasen, die durch Induktion von Typ-1-Interferonen und anderen Zytokinen eine ausschlaggebende Rolle bei der angeborenen Immunantwort spielen. Diese Kinasen werden als Antwort auf eine virale/bakterielle Infektion stimuliert. Zur Immunantwort auf virale und bakterielle Infektionen gehört die Bindung von Antigenen wie bakteriellem Lipopolysaccharid (LPS), viraler doppelsträngiger RNA (dsRNA) an Toll-like Rezeptoren, daran anschließend Aktivierung des TBK1-Weges. Aktiviertes TBK1 und IKKε phosphorylieren IRF3 und IRF7, was die Dimerisierung und Kerntranslokation dieser Interferon regulierenden Transkriptionsfaktoren auslöst, was letztendlich eine Signalkaskade induziert, die zur IFN-Produktion führt.

Kürzlich wurden IKKε und TBK1 auch mit Krebs in Zusammenhang gebracht. Es wurde gezeigt, daß IKKε mit aktiviertem MEK zur Transformation menschlicher Zellen kooperiert. Außerdem wird IKKε häufig in Brustkrebs-Zellinien und von Patienten stammenden Tumoren amplifiziert/überexprimiert. TBK1 wird unter hypoxischen Bedingungen induziert und in vielen soliden Tumoren in signifikanter Höhe exprimiert. Des weiteren ist TBK1 erforderlich, um onkogene Ras-Transformation zu unterstützen, und TBK1-Kinaseaktivität ist in transformierten Zellen erhöht und für ihr Überleben in Kultur erforderlich. Ebenso wurde gefunden, daß TBK1- und NF-kB-Signalgebung in KRAS-mutierten Tumoren wesentlich sind. TBK1 wurde als ein synthetischer letaler Partner des onkogenen KRAS identifiziert.

### Lit.:

Y.-H.Ou et al., Molecular Cell 41, 458-470, 2011;
D.A. Barbie et al., Nature, 1-5, 2009.

In der WO 2011/046970 A1 wird die Verwendung von TBK1- und/oder IKKε - Inhibitoren zur Behandlung von verschiedenen Krankheiten beschrieben, wie rheumatoide Arthritis (RA), systemischer Lupus erythematosus (SLE), Sjörgrens Syndrom, Aicardi-Goutieres Syndrom Lupus Chilblain, retinale Vasculopathie und cerebrale Leukodystrophie (RVCL), systemische Sclerosis, Myositis, Psoriasis, chronisch obstruktive pulmonare Krankheit (CPD), endzündliche Darmkrankheit (IBD), Fettsucht, Insulinresistenz, Typ 2 Diabetes (NIDDM), metabolisches Syndrom, Krebserkrankungen,

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt.
Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Der Ausdruck "Methode" bezeichnet Arbeitsweisen, Mittel, Techniken und Prozeduren, um eine gegebene Aufgabe zu erfüllen, darunter diejenigen Arbeitsweisen, Mittel, Techniken und Prozeduren, die dem Fachmann auf chemischem, pharmakologischem, biologischem, biochemischem und medizinischem Gebiet entweder bekannt sind oder von ihm leicht aus bekannten Arbeitsweisen, Mitteln, Techniken und Prozeduren entwickelt werden können, ohne jedoch hierauf beschränkt zu sein.

Der Ausdruck "Verabreichung", wie er hier verwendet wird, bezeichnet eine Methode, um eine Verbindung der vorliegenden Erfindung und eine Zielkinase so zusammenzubringen, daß die Verbindung die Enzymaktivität der Kinase entweder direkt, d.h. durch Wechselwirkung mit der Kinase selber, oder indirekt, d.h. durch Wechselwirkung mit einem anderen Molekül, von dem die katalytische Aktivität der Kinase abhängt, beeinflussen kann. Wie hier verwendet, kann Verabreichung entweder in vitro, d.h. im Reagenzglas, oder in vivo, d.h. in Zellen oder Geweben eines lebenden Organismus, erfolgen.

Der Ausdruck "Behandeln" umfaßt hier Außerkraftsetzen, weitgehendes Hemmen, Verlangsamen oder Umkehren des Fortschreitens einer Krankheit oder Störung, weitgehendes Verbessern der klinischen Symptome einer Krankheit oder Störung oder weitgehendes Verhindern des Auftretens der klinischen Symptome einer Krankheit oder Störung.

Der Ausdruck "Verhindern" bezeichnet hier eine Methode, um einen Organismus dagegen zu blockieren, daß er eine Störung oder Krankheit überhaupt erwirbt.

Für eine beliebige in dieser Erfindung verwendete Verbindung kann eine therapeutisch wirksame Menge, die hier auch als therapeutisch wirksame Dosis bezeichnet wird, kann zunächst anhand von Zellkultur-Assays berechnet werden. Beispielsweise kann in Tiermodellen eine Dosis formuliert werden, um einen Kreislaufkonzentrationsbereich zu erreichen, der die IC50 oder die IC100 umfaßt, wie sie in Zellkulturen ermittelt wurden. Diese Information kann verwendet werden, um brauchbare Dosen für den Menschen genauer zu bestimmen. Anfangsdosierungen können auch aus In-vivo-Daten berechnet werden. Anhand dieser Anfangsrichtlinien könnte ein Durchschnittsfachmann eine wirksame Dosierung für den Menschen bestimmen.

Außerdem können die Toxizität und die therapeutische Wirksamkeit der hier beschriebenen Verbindungen nach pharmazeutischen Standardprozeduren an Zellkulturen oder Versuchstieren bestimmt werden, indem man z.B. die LD50 und die ED50 bestimmt. Das Dosisverhältnis zwischen toxischer und therapeutischer Wirkung ist der therapeutische Index, der als das Verhältnis zwischen LD50 und ED50 ausgedrückt werden kann. Verbindungen, die einen hohen therapeutischen Index aufweisen, sind bevorzugt. Die aus diesen Zellkultur-Assays und Tierversuchen erhaltenen Daten können verwendet werden, um einen Dosierungsbereich zu formulieren, der für die Verwendung am Menschen nicht toxisch ist. Die Dosierung solcher Verbindungen liegt vorzugsweise in Konzentrationsbereichen im Blutkreislauf, die die ED50 mit geringer oder keiner Toxizität umfassen. Innerhalb dieses Bereiches kann die Dosierung in Abhängigkeit von der eingesetzten Dosierungsform und dem verwendeten Verabreichungsweg variieren. Die genaue Formulierung, der Verabreichungsweg und die Dosierung können vom einzelnen Arzt unter Berücksichtigung des Zustandes des Patienten gewählt werden (siehe z.B. Fingl et al., 1975, in: The Pharmacological Basis of Therapeutics, Kapitel 1, Seite 1).

Dosierungsmenge und -interval können individuell eingestellt werden, um für Plasmaspiegel der Wirkverbindung zu sorgen, die ausreichen, um eine therapeutische Wirkung zu erhalten. Übliche Patientendosierungen für orale Verabreichung liegen im Bereich von etwa 50-2000 mg/kg/Tag, im allgemeinen von etwa 100-1000 mg/kg/Tag, vorzugsweise von etwa 150-700 mg/kg/Tag und insbesondere bevorzugt von etwa 250-500 mg/kg/Tag.

Vorzugsweise erreicht man therapeutisch wirksame Serumspiegel durch Verabreichen mehrerer Dosen pro Tag. Bei lokaler Verabreichung oder selektiver Aufnahme steht die wirksame lokale Konzentration des Medikamentes möglicherweise nicht mit der Plasmakonzentration in Beziehung. Der Fachmann wird in der Lage sein, therapeutisch wirksame lokale Dosierungen ohne übermäßiges Experimentieren zu optimieren.

Bevorzugte Krankheiten oder Störungen, für deren Vorbeugung, Behandlung und/oder Untersuchung die hier beschriebenen Verbindungen brauchbar sein können, sind zellproliferative Störungen, insbesondere Krebs, wie Papillom, Blastogliom, Kaposi-Sarkom, Melanom, Lungenkrebs, Eierstockkrebs, Prostatakrebs, Plattenepithelkarzinom, Astrozytom, Kopfkrebs, Halskrebs, Hautkrebs, Leberkrebs, Blasenkrebs, Brustkrebs, Lungenkrebs, Gebärmutterkrebs, Prostatakrebs, Hodenkarzinom, Kolorektalkrebs, Schilddrüsenkrebs, Bauchspeicheldrüsenkrebs, Magenkrebs, hepatozelluläres Karzinom, Leukämie, Lymphom, Morbus Hodgkin und Burkitt-Krankheit, ohne hierauf beschränkt zu sein.

### STAND DER TECHNIK

Andere Benzonitrilderivate sind in der WO 2011/046970 A1 und in WO 2012/010826 A1 als TBK1- und/oder IKKε-Inhibitoren beschrieben.

Weitere heterocyclische Derivate und deren Verwendung als Antitumormittel wurden in der WO 2007/129044 beschrieben.

Weitere Pyridin- und Pyrazinderivate wurden in der Verwendung für die Behandlung von Krebs in der WO 2009/053737 und für die Behandlung von anderen Krankheiten in der WO 2004/055005 beschrieben.

Weitere heterocyclische Derivate wurden als IKKε-Hemmer in der WO 2009/122180 offenbart.

Pyrrolopyrimidine wurden als IKKε- und TBK1-Hemmer in der WO 2010/100431 beschrieben.

Pyrimidinderivate wurden als IKKε- und TBK1-Hemmer in der WO 2009/030890 beschrieben.

### ZUSAMMENFASSUNG DER ERFINDUNG

Die Erfindung betrifft einzelne Verbindungen gemäß Anspruch 1 umfaßt von der Formel I 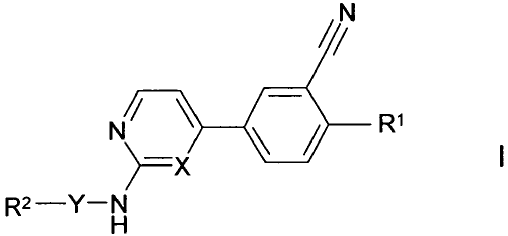 worin
- X: CH oder N,
- Y: Het²-diyl,
- R¹: O(CH₂)ₙHet¹, NH(CH₂)ₙHet¹, OA, NHA, NA₂, O(CH₂)ₙCyc oder NH(CH₂)ₙCyc,
- R²: H, A, Ar¹, (CH₂)ₙHet³, CN, (CH₂)ₙCyc, CONH₂, COOA, (CH₂)ₙOH, (CH₂)ₙOA, (CH₂)ₙNH₂, (CH₂)ₙNHA oder (CH₂)ₙNA₂,
- Ar¹: unsubstituiertes oder ein-, zwei- oder dreifach durch Hal, A, OH, OA, COOH, COOA, CN, CONH₂, NHSO₂A und/oder SO₂A substituiertes Phenyl,
- Het¹: unsubstituiertes oder einfach durch OH, COOA, CONH₂, COA und/oder A substituiertes Dihydropyrrolyl, Pyrrolidinyl, Azetidinyl, Tetrahydroimidazolyl, Dihydropyrazolyl, Tetrahydropyrazolyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl oder Piperazinyl,
- Het²: unsubstituiertes oder einfach durch Hal, A, OH, =O, OA, CN, COOA, COOH, CONH₂ und/oder NHCOA substituiertes Furyl, Thienyl, Pyrrolyl, Imidazolyl, Pyrazolyl, Oxazolyl, Isoxazolyl, Thiazolyl, Triazolyl, Pyridyl, Pyrimidyl, Pyridazinyl, Pyrazinyl, Indolyl, Isoindolyl, Benzimidazolyl, Indazolyl, Chinolyl, 1,3-Benzodioxolyl, Benzothiophenyl, Benzofuranyl, Imidazopyridyl, 5,6,7,8-Tetrahydro-pyrido[4,3-d]pyrimidin-2-yl oder Furo[3,2-b]pyridyl,
- Het³: unsubstituiertes oder ein- oder zweifach durch Hal, A, OH, OA, CN, COOA, COOH, CONH₂, CONHA, CONA₂, COA, COCH₂NH₂, COCH₂NHA, COCH₂NA₂, (CH₂)ₙCyc und/oder NHCOA substituiertes Dihydropyrrolyl, Pyrrolidinyl, Azetidinyl, Tetrahydroimidazolyl, Tetrahydrofuranyl, Dihydropyrazolyl, Tetrahydropyrazolyl, Dihydropyridyl, Tetrahydropyridyl, Piperidinyl, Morpholinyl, Hexahydropyridazinyl, Hexahydropyrimidinyl, [1,3]Dioxolanyl, Dihydropyranyl, Tetrahydropyranyl, Piperazinyl, Furyl, Thienyl, Pyrrolyl, Imidazolyl, Pyrazolyl, Oxazolyl, Isoxazolyl, Thiazolyl, Triazolyl, Pyridyl, Pyrimidyl, Pyridazinyl, Indolyl, Isoindolyl, Benzimidazolyl, Indazolyl, Chinolyl, 1,3-Benzodioxolyl, Benzothiophenyl, Benzofuranyl, Imidazopyridyl oder Furo[3,2-b]pyridyl,
- A: unverzweigtes oder verzweigtes Alkyl mit 1-10 C-Atomen, worin eine oder zwei nicht benachbarte CH- und/oder CH₂-Gruppen durch N-, O- und/oder S-Atomen ersetzt sein können und/oder auch 1-7 H-Atome durch F und/oder Cl ersetzt sein können,
- Cyc: unsubstituiertes oder einfach durch CN, (CH₂)ₙOH oder A substituiertes cyclisches Alkyl mit 3, 4, 5, 6 oder 7 C-Atomen,
- Hal: F, Cl, Br oder I,
- n: 0, 1, 2, 3 oder 4,
bedeuten,
sowie ihre pharmazeutisch verwendbaren Salze, Tautomere und Stereoisomere, einschließlich deren Mischungen in allen Verhältnissen.

Der Anmeldungsgegenstand bezieht sich auf die Definition der Ansprüche; jede Offenbarung, die über den Umfang der Ansprüche hinausgeht, dient nur zu Informationszwecken.

Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Gegenstand der Erfindung sind natürlich auch die Solvate der Salze.

Unter pharmazeutisch verwendbaren Derivaten versteht man z.B. die Salze der erfindungsgemäßen Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden. Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.
Gegenstand der Erfindung ist auch die Verwendung von Mischungen der Verbindungen der Formel I gemäß Anspruch 1, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Offenbarung ist ein Verfahren zur Herstellung von Verbindungen der Formel I sowie ihrer pharmazeutisch verwendbaren Salze, Tautomere und Stereoisomere, dadurch gekennzeichnet, daß man
a) eine Verbindung der Formel II

   R²-Y-NH₂ II

   worin Y und R² die in Anspruch 1 angegebenen Bedeutungen haben,
   mit einer Verbindung der Formel III 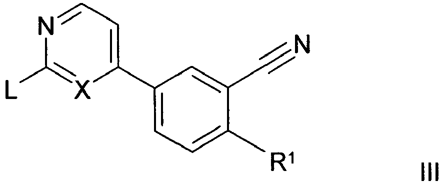 worin X und R¹ die in Anspruch 1 angegebenen Bedeutungen haben und L F, Cl, Br oder I bedeutet,
   umsetzt,
   und/oder eine Base oder Säure der Formel I in eines ihrer Salze umwandelt.

Vor- und nachstehend haben die Reste R¹, R², X und Y die bei der Formel I angegebenen Bedeutungen, sofern nicht ausdrücklich etwas anderes angegeben ist.

A bedeutet Alkyl, ist unverzweigt (linear) oder verzweigt und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
In A können auch eine oder zwei CH- und/oder CH₂-Gruppen durch N, O- oder S-Atome ersetzt sein. So bedeutet A z.B. auch 2-Methoxyethyl.
Besonders bevorzugt bedeutet A unverzweigtes oder verzweigtes Alkyl mit 1-8 C-Atomen, worin eine oder zwei nicht benachbarte CH- und/oder CH₂-Gruppen durch N- und/oder O-Atome ersetzt sein können und/oder auch 1-7 H-Atome durch F ersetzt sein können.

Ar¹ bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl. Ar¹ bedeutet besonders bevorzugt unsubstituiertes oder ein-, zwei- oder dreifach durch A substituiertes Phenyl.

Het¹ bedeutet vorzugsweise unsubstituiertes oder einfach durch COA substituiertes Pyrrolidinyl, Piperidinyl, Morpholinyl oder Tetrahydropyranyl.
Het² bedeutet vorzugsweise unsubstituiertes oder einfach durch =O oder OA substituiertes Thienyl, Pyrazolyl, Oxazolyl, Isoxazolyl, Pyridyl, Pyrazinyl, Pyridazinyl, Thiazolyl, Pyrimidyl, Indolyl, 5,6,7,8-Tetrahydro-pyrido[4,3-d]pyrimidin-2-yl oder Benzofuranyl.
Het³ bedeutet vorzugsweise unsubstituiertes oder einfach durch A substituiertes Pyrrolidinyl, Azetidinyl, Tetrahydrofuranyl, Dihydropyranyl, Tetrahydropyranyl, Dihydropyridyl, Tetrahydropyridyl, Piperidinyl, Piperazinyl, Morpholinyl, Furyl, Thienyl, Pyrazolyl, Benzofuranyl oder Pyridyl.

Hal bedeutet vorzugsweise F, Cl oder Br, aber auch I, besonders bevorzugt F oder Cl. X bedeutet vorzugsweise CH.

Für die gesamte Offenbarung gilt, daß sämtliche Reste, die mehrfach auftreten, gleich oder verschieden sein können, d.h. unabhängig voneinander sind.
Die Verbindungen der Formel I gemäß Anspruch 1 können ein oder mehrere chirale Zentren besitzen und daher in verschiedenen stereoisomeren Formen vorkommen. Die Formel I umschließt alle diese Formen.
Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.
Verbindungen der Formel I können vorzugsweise erhalten werden, indem man Verbindungen der Formel II mit einer Verbindung der Formel III umsetzt.
Die Verbindungen der Formel II und der Formel III sind in der Regel bekannt. Sind sie neu, so können sie aber nach an sich bekannten Methoden hergestellt werden. Die Umsetzung erfolgt vorzugsweise unter Buchwald-Hartwig-Bedingungen, die dem Fachmann bekannt sind.
Die Reaktionszeit liegt je nach den angewendeten Bedingungen zwischen einigen Minuten und 14 Tagen, die Reaktionstemperatur zwischen etwa -10° und 160°, normalerweise zwischen 20° und 150°, besonders bevorzugt zwischen 80° und 150°C.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether, Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Dioxan.

In den Verbindungen der Formel III bedeutet L vorzugsweise Cl, Br oder I, besonders bevorzugt Cl.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.
Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

Alkylierungen am Stickstoff erfolgen unter Standardbedingungen, wie sie dem Fachmann bekannt sind.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I gemäß Anspruch 1 werden größtenteils konventionell hergestellt. Sofern die Verbindung der Formel I gemäß Anspruch 1 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Kalziumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Kalzium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium, sowie die Erdalkalimetalsalze Kalzium und Magnesium. Zu Salzen der Verbindungen der Formel I gemäß Anspruch 1, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I gemäß Anspruch 1 mit Metallen oder Aminen wie Alkalimetallen und Erdalkalimetallen oder organischen Aminen gebildet. Bevorzugte Metalle sind Natrium, Kalium, Magnesium und Kalzium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I gemäß Anspruch 1 und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie ihre pharmazeutisch verwendbaren Salze, Tautomere und Stereoisomere lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I gemäß Anspruch 1 sowie ihre pharmazeutisch verwendbaren Salze, Tautomere und Stereoisomere können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Poly-epsilon-caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I gemäß Anspruch 1 hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandeln den Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, oben erwähnten Krankheitszustände geeignet sind. Gegenstand der Erfindung sind ferner Arzneimittel enthaltend mindestens eine Verbindung der Formel I gemäß Anspruch 1 und/oder sowie ihre pharmazeutisch verwendbaren Salze, Tautomere und Stereoisomere, einschließlich deren Mischungen in allen Verhältnissen, und mindestens einen weiteren Arzneimittelwirkstoff.

Gegenstand der Offenbarung ist auch ein Set (Kit), bestehend aus getrennten Packungen von
(a) einer wirksamen Menge an einer Verbindung der Formel I gemäß Anspruch 1 und/oder ihre pharmazeutisch verwendbaren Salze, Tautomere und Stereoisomere, einschließlich deren Mischungen in allen Verhältnissen, und
(b) einer wirksamen Menge eines weiteren Arzneimittelwirkstoffs.
Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihre pharmazeutisch verwendbaren Salze, Tautomere und Stereoisomere, einschließlich deren Mischungen in allen Verhältnissen, und einer wirksamen Menge eines weiteren Arzneimittelwirkstoffs gelöst oder in lyophilisierter Form vorliegt.

### Isotope

Es ist weiterhin vorgesehen, daß eine Verbindung der Formel I gemäß Anspruch 1 isotopenmarkierte Formen davon umfaßt. Eine isotopenmarkierte Form einer Verbindung der Formel I gemäß Anspruch 1 ist mit dieser Verbindung bis auf die Tatsache, daß eines oder mehrere Atome der Verbindung durch ein Atom bzw. Atome mit einer Atommasse oder Massenzahl ersetzt wurden, die sich von der Atommasse oder Massenzahl des Atoms, das üblicherweise natürlich vorkommt, unterscheidet, identisch. Zu den Isotopen, die leicht im Handel erhältlich sind und in eine Verbindung der Formel I gemäß Anspruch 1 nach gut bekannten Verfahren eingebaut werden können, zählen zum Beispiel Isotope von Wasserstoff, Kohlenstoff, Stickstoff, Sauerstoff, Phosphor, Fluor und Chlor, z.B. ²H, ³H, ¹³C, ¹⁴C, ¹⁵N, ¹⁸O, ¹⁷O, ³¹P, ³²P, ³⁵S, ¹⁸F bzw. ³⁶Cl. Eine Verbindung der Formel I gemäß Anspruch 1, oder jeweils ein pharmazeutisch unbedenkliches Salz davon, die eines oder mehrere der oben genannten Isotope und/oder andere Isotope von anderen Atomen enthält, ist als Bestandteil der vorliegenden Erfindung vorgesehen. Eine isotopenmarkierte Verbindung der Formel I gemäß Anspruch 1 läßt sich auf vielerlei nützliche Art verwenden. Zum Beispiel eignet sich eine isotopenmarkierte Verbindung der Formel I gemäß Anspruch 1, in die z.B. ein Radioisotop wie ³H oder ¹⁴C eingebaut worden ist, für Assays zur Verteilung des Arzneistoffs und/oder Substratgewebes. Diese Radioisotope, d.h. Tritium (³H) und Kohlenstoff-14 (¹⁴C), sind aufgrund ihrer einfachen Herstellung und ausgezeichneten Nachweisbarkeit besonders bevorzugt. Der Einbau schwererer Isotope, z.B. Deuterium (²H), in eine Verbindung der Formel I gemäß Anspruch 1 weist therapeutische Vorteile aufgrund der höheren Stabilität dieser isotopenmarkierten Verbindung im Metabolismus auf. Höhere Stabilität in Metabolismus bedeutet unmittelbar eine erhöhte Halbwertszeit in vivo oder niedrigere Dosierungen, was unter den meisten Umständen eine bevorzugte Ausführungsform der vorliegenden Erfindung darstellen würde. Eine isotopenmarkierte Verbindung der Formel I gemäß Anspruch 1 läßt sich üblicherweise durch Durchführung der in den Syntheseschemata und der damit in Zusammenhang stehenden Beschreibung, im Beispielteil und im Herstellungsteil im vorliegenden Text offenbarten Vorgehensweisen herstellen, wobei ein nicht isotopenmarkierter Reaktionspartner durch einen leicht verfügbaren isotopenmarkierten Reaktionspartner ersetzt wird.

Zur Manipulation des oxidativen Metabolismus der Verbindung über den primären kinetischen Isotopeneffekt kann auch Deuterium (²H) in eine Verbindung der Formel I gemäß Anspruch 1 eingebaut werden. Beim primären kinetischen Isotopeneffekt handelt es sich um eine Veränderung der Geschwindigkeit einer chemischen Reaktion aufgrund des Austausches isotopischer Kerne, was wiederum durch die Änderung der für die Bildung kovalenter Bindungen im Anschluß an diesen isotopischen Austausch erforderlichen Grundzustandsenergien verursacht wird. Der Austausch eines schwereren Isotops führt üblicherweise zu einer Erniedrigung der Grundzustandsenergie für eine chemische Bindung und verursacht so eine Verringerung der Geschwindigkeit bei einem geschwindigkeitslimitierenden Bindungsbruch. Findet der Bindungsbruch an bzw. in der Nähe einer Sattelpunktregion entlang der Koordinate einer Reaktion mit mehreren Produkten statt, so können sich die Produktverteilungsverhältnisse stark ändern. Zur Erläuterung: Wird Deuterium an ein Kohlenstoffatom in einer nichtaustauschbaren Position gebunden, so sind Geschwindigkeitsunterschiede von k_{M}/k_{D} = 2-7 typisch. Wird dieser Geschwindigkeitsunterschied erfolgreich auf eine oxidationsanfällige Verbindung der Formel I gemäß Anspruch 1 angewandt, so kann sich dadurch das Profil dieser Verbindung in vivo drastisch ändern und zu verbesserten pharmakokinetischen Eigenschaften führen.

Bei der Entdeckung und Entwicklung von Therapeutika versucht der Fachmann, pharmakokinetische Parameter zu optimieren und gleichzeitig wünschenswerte In-vitro-Eigenschaften beizubehalten. Man kann vernünftig annehmen, daß viele Verbindungen mit schlechten pharmakokinetischen Profilen gegenüber dem oxidativen Metabolismus anfällig sind. Aus derzeitig verfügbaren In-vitro-Assays mit Lebermikrosomen erhält man wertvolle Informationen über den Verlauf dieses oxidativen Metabolismus, aufgrund dessen wiederum deuterierte Verbindungen der Formel I mit einer verbesserten Stabilität durch Resistenz gegenüber einem derartigen oxidativen Metabolismus rational gestaltet werden können. So gelangt man zu wesentlichen Verbesserungen der pharmakokinetischen Profile der Verbindungen der Formel I gemäß Anspruch 1, die sich quantitativ als erhöhte Invivo-Halbwertszeit (T/2), Konzentration bei maximaler therapeutischer Wirkung (Cmax), Fläche unter der Dosis-Wirkungskurve (AUC) sowie F und als verringerte Clearance, Dosis und Materialkosten ausdrücken lassen.

Zur Veranschaulichung des Obigen soll folgendes dienen: eine Verbindung der Formel I gemäß Anspruch 1 mit mehrfachen potentiellen Angriffsstellen für den oxidativen Metabolismus, z.B. Wasserstoffatome an einem Benzylrest und Wasserstoffatome, die an ein Stickstoffatom gebunden sind, wird als Reihe von Analogen hergestellt, in denen verschiedene Kombinationen von Wasserstoffatomen durch Deuteriumatome ersetzt werden, so daß einige, die meisten oder alle dieser Wasserstoffatome durch Deuteriumatome ersetzt sind. Durch Bestimmungen der Halbwertszeit gelangt man zu einer günstigen und genauen Bestimmung, wie sehr sich die Verbesserung der Widerstandsfähigkeit gegenüber oxidativen Metabolismen verbessert hat. Auf diese Weise wird bestimmt, daß aufgrund eines derartigen Austausches von Wasserstoff gegen Deuterium die Halbwertszeit der Ausgangsverbindung um bis zu 100% verlängert werden kann.

Der Austausch von Wasserstoff gegen Deuterium in einer Verbindung der Formel I gemäß Anspruch 1 läßt sich auch dazu verwenden, um zu einer günstigen Änderung des Stoffwechselproduktspektrums der Ausgangsverbindung zwecks Verringerung oder Ausschluß von unerwünschten toxischen Stoffwechselprodukten zu gelangen. Entsteht zum Beispiel ein toxisches Stoffwechselprodukt aufgrund der Spaltung einer oxidativen Kohlenstoff-Wasserstoff (C-H)-Bindung kann vernünftigerweise angenommen werden, daß das deuterierte Analog die Produktion des unerwünschten Stoffwechselprodukts wesentlich verringert oder ausschließt, sogar dann, wenn es sich bei der jeweiligen Oxidation nicht um einen geschwindigkeitsbestimmenden Schritt handelt. Weitere Informationen zum Stand der Technik in bezug auf den Austausch von Wasserstoff gegen Deuterium finden sich z.B. bei Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al., Biochemistry 33(10), 2927-2937, 1994, und Jarman et al., Carcinogenesis 16(4), 683-688, 1993.

### VERWENDUNG

Die Erfindung betrifft die Verbindungen der Formel I gemäß Anspruch 1 zur Verwendung für die Behandlung von Krebs, septischem Schock, primärem Offenwinkelglaukom (POAG), Hyperplasie, rheumatoider Arthritis, Psoriasis, Atherosklerose, Retinopathie, Osteoarthritis, Endometriose, chronischer Entzündung und/oder neurodegenerativen Erkrankungen wie Morbus Alzheimer.

Die Erfindung betrifft weiterhin die Verbindungen der Formel I zur Verwendung für die Behandlung von Krebs, septischem Schock, primärem Offenwinkelglaukom (POAG), Hyperplasie, Atherosklerose, Retinopathie, Osteoarthritis, Endometriose, chronischer Entzündung, neurodegenerativen Erkrankungen, rheumatoide Arthritis (RA), systemischer Lupus erythematosus (SLE), Sjörgrens Syndrom, Aicardi-Goutieres Syndrom Lupus Chilblain, retinale Vasculopathie, cerebrale Leukodystrophie (RVCL), systemische Sklerosis, Myositis, Psoriasis, chronisch obstruktive pulmonare Krankheit (CPD), endzündliche Darmkrankheit (IBD), Fettsucht, Insulinresistenz, Typ 2 Diabetes (NIDDM) und/oder metabolisches Syndrom.

Die vorliegenden Verbindungen eignen sich als pharmazeutische Wirkstoffe für Säugetiere, insbesondere für den Menschen, bei der Behandlung und Bekämpfung von Krebserkrankungen und Entzündungserkrankungen.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln wie Anti-IgM zu ermöglichen, eine Zellantwort wie Expression eines Oberflächenmarkers zu induzieren, gewöhnlich zwischen ungefähr einer Stunde und einer Woche. Zum Testen in vitro können kultivierte Zellen aus Blut oder einer Biopsieprobe verwendet werden. Die Menge an exprimiertem Oberflächenmarker wird durch Durchflußzytometrie beurteilt, wobei spezielle Antikörper verwendet werden, die den Marker erkennen.
Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszenz nachweisbar (Ross et al., 2002, Biochem. J.).

Die vorliegende Erfindung umfasst die Verwendung der Verbindungen der Formel I gemäß Anspruch 1 und/oder ihre physiologisch unbedenklichen Salze, Tautomere und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom Darmkrebs. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebenfalls umfasst ist die Verwendung der Verbindungen der Formel I und/oder ihre physiologisch unbedenklichen Salze, Tautomere und Solvate zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt sind die Verbindungen zur Verwendung zur Behandlung einer Krankheit, wobei die Krebskrankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt sind die Verbindungen zur Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung sind weiterhin die erfindungsgemäßen Verbindungen zur Verwendung zur Behandlung von Knochen-Pathologien, wobei die Knochenpathologie aus der Gruppe Osteosarkom, Osteoarthritis und Rachitis stammt.

Die Verbindungen der Formel I gemäß Anspruch 1 können auch gemeinsam mit anderen gut bekannten Therapeutika, die aufgrund ihrer jeweiligen Eignung für das behandelte Leiden ausgewählt werden, verabreicht werden.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)-ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenyl-retinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin-(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]-chinolin-10,13(9H,15H)-dion, Lurtotecan, 7-[2-(N-Isopropylamino)ethyl]-(20S)-camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carbonsäureamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydro-furo(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo-[g]isochinolin-5,10-dion, 5-(3-Aminopropylamino)-7,10-dihydroxy-2-(2-hydroxyethyl-aminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carbonsäureamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxy-methyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson |
| | Tetraplatin | Matthey) |
| | Ormiplatin | BBR-3464 (Hoffrnann-La |
| | Iproplatin | Roche) |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La |
| | Idatrexate | Roche) |
| | | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour- |
| | 7-Ethyl-10-hydroxycamptothecin | Ipsen) |
| | Topotecan | TAS-103 (Taiho) |
| | Dexrazoxanet (TopoTarget) | Elsamitrucin (Spectrum) |
| | Pixantron (Novuspharrna) | J-107088 (Merck & Co) |
| | Rebeccamycin-Analogon | BNP-1350 (BioNumerik) |
| | (Exelixis) | CKD-602 (Chong Kun |
| | BBR-3576 (Novuspharrna) | Dang) |
| | | KW-2170 (Kyowa Hakko) |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat |
| | Therarubicin | (Blenoxan) |
| | Idarubicin | Bleomycinsäure |
| | Rubidazon | Bleomycin A |
| | Plicamycinp | Bleomycin B |
| | Porfiromycin | Mitomycin C |
| | Cyanomorpholinodoxorubicin | MEN-10755 (Menarini) |
| | Mitoxantron (Novantron) | GPX-100 (Gem |
| | | Pharmaceuticals) |
| | | |
| Antimitotische Mittel | Paclitaxel | SB 408075 |
| | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | Cemadotin (BASF) | ER-86526 (Eisai) |
| | RPR 109881A (Aventis) | Combretastatin A4 (BMS) |
| | TXD 258 (Aventis) | Isohomohalichondrin-B |
| | Epothilon B (Novartis) | (PharmaMar) |
| | T 900607 (Tularik) | ZD 6126 (AstraZeneca) |
| | T 138067 (Tularik) | PEG-Paclitaxel (Enzon) |
| | Cryptophycin 52 (Eli Lilly) | AZ10992 (Asahi) |
| | Vinflunin (Fabre) | IDN-5109 (Indena) |
| | Auristatin PE (Teikoku | AVLB (Prescient |
| | Hormone) | NeuroPharma) |
| | BMS 247550 (BMS) | Azaepothilon B (BMS) |
| | BMS 184476 (BMS) | BNP- 7787 (BioNumerik) |
| | BMS 188797 (BMS) | CA-4-Prodrug (OXiGENE) |
| | Taxoprexin (Protarga) | Dolastatin-10 (NrH) CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase- | Pemetrexed (Eli Lilly) | Nolatrexed (Eximias) |

| Inhibitoren | ZD-9331 (BTG) | CoFactor™ (BioKeys) |
|---|---|---|
| | | |
| DNA-Antagonisten | Trabectedin (PharmaMar) Glufosfamid (Baxter International) Albumin + 32P (Isotope Solutions) Thymectacin (NewBiotics) Edotreotid (Novartis) | Mafosfamid (Baxter International) Apaziquon (Spectrum Pharmaceuticals) O6-Benzylguanin (Paligent) |
| | | |
| Farnesyltransferase-Inhibitoren | Arglabin (NuOncology Labs) lonafarnib (Schering-Plough) BAY-43-9006 (Bayer) | Tipifarnib (Johnson & Johnson) Perillylalkohol (DOR BioPharma) |
| | | |
| Pumpen-Inhibitoren | CBT-1 (CBA Pharma) Tariquidar (Xenova) MS-209 (Schering AG) | Zosuquidar-Trihydrochlorid (Eli Lilly) Biricodar-Dicitrat (Vertex) |
| | | |
| Histonacetyltransferase-Inhibitoren | Tacedinalin (Pfizer) SAHA (Aton Pharma) MS-275 (Schering AG) | Pivaloyloxymethylbutyrat (Titan) Depsipeptid (Fujisawa) |
| | | |
| Metalloproteinase-Inhibitoren Ribonucleosidredukt ase-Inhibitoren | Neovastat (Aeterna Laboratories) Marimastat (British Biotech) Galliummaltolat (Titan) Triapin (Vion) | CMT -3 (CollaGenex) BMS-275291 (Celltech) Tezacitabin (Aventis) Didox (Molecules for Health) |
| | | |
| TNF-alpha-Agonisten / Antagonisten | Virulizin (Lorus Therapeutics) CDC-394 (Celgene) | Revimid (Celgene) |
| | | |
| Endothelin-A-Rezeptor-Antaqonisten | Atrasentan (Abbot) ZD-4054 (AstraZeneca) | YM-598 (Yamanouchi) |
| | | |
| Retinsäurerezeptor-Agonisten | Fenretinid (Johnson & Johnson) LGD-1550 (Ligand) | Alitretinoin (Ligand) |
| | | |
| Immunmodulatoren | Interferon Oncophage (Antigenics) GMK (Progenics) Adenokarzinom-Impfstoff (Biomira) CTP-37 (AVI BioPharma) | Dexosom-Therapie (Anosys) Pentrix (Australian Cancer Technology) JSF-154 (Tragen) Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL | MGV (Progenics) |
| | Immuno) | !3-Alethin (Dovetail) |
| | Melanom-Impfstoff (CTL | CLL-Thera (Vasogen) |
| | Immuno) | |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische Mittel | Talaporfin (Light Sciences) | Pd-Bacteriopheophorbid |
| | Theralux (Theratechnologies) | (Yeda) |
| | Motexafin-Gadolinium | Lutetium-Texaphyrin |
| | (Pharmacyclics) | (Pharmacyclics) Hypericin |
| | | |
| Tyrosinkinase- | Imatinib (Novartis) | Kahalid F (PharmaMar) |
| Inhibitoren | Leflunomid (Sugen/Pharmacia) | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | 2C4 (Genentech) |
| | PKI166 (Novartis) | MDX-447 (Medarex) |
| | GW2016 (GlaxoSmithKline) | ABX-EGF (Abgenix) |
| | EKB-509 (Wyeth) | IMC-1C11 (ImClone) |
| | EKB-569 (Wyeth) | |
| Verschiedene Mittel | SR-27897 (CCK-A-Inhibitor, | BCX-1777 (PNP-Inhibitor, |
| | Sanofi-Synthelabo) Tocladesin (cyclisches-AMP-Agonist, Ribapharm) Alvocidib (CDK-Inhibitor, Aventis) CV-247 (COX-2-Inhibitor, Ivy Medical) P54 (COX-2-Inhibitor, Phytopharm) CapCell™ (CYP450-Stimulans, Bavarian Nordic) GCS-IOO (gal3-Antagonist, GlycoGenesys) G17DT-Immunogen (Gastrin-Inhibitor, Aphton) Efaproxiral (Oxygenator, Allos Therapeutics) PI-88 (Heparanase-Inhibitor, Progen) Tesmilifen (Histamin-Antagonist, YM BioSciences) Histamin (Histamin-H2-Rezeptor- Agonist, Maxim) Tiazofurin (IMPDH-Inhibitor, Ribapharm) Cilengitid (Integrin-Antagonist, Merck KGaA) SR-31747 (IL-1-Antagonist, Sanofi-Synthelabo) CCI-779 (mTOR-Kinase-Inhibitor, Wyeth) Exisulind (PDE-V-Inhibitor, Cell Pathways) CP-461 (PDE-V-Inhibitor, Cell Pathways) AG-2037 (GART-Inhibitor, Pfizer) WX-UK1 (Plasminogenaktivator-Inhibitor, Wilex) PBI-1402 (PMN-Stimulans, ProMetic LifeSciences) Bortezomib (Proteasom-Inhibitor, Millennium) SRL-172 (T-Zell-Stimulans, SR Pharma) TLK-286 (Glutathion-S-Transferase-Inhibitor, Telik) | BioCryst) Ranpirnase (Ribonuclease-Stimulans, Alfacell) Galarubicin (RNA-Synthese-Inhibitor, Dong-A) Tirapazamin (Reduktionsmittel, SRI International) N-Acetylcystein (Reduktionsmittel, Zambon) R-Flurbiprofen (NFkappaB-Inhibitor, Encore) 3CPA (NF-kappaB-Inhibitor, Active Biotech) Seocalcitol (Vitamin-D-Rezeptor-Agonist, Leo) 131-I-TM-601 (DNA-Antagonist, TransMolecular) Eflornithin (ODC-Inhibitor, ILEX Oncology) Minodronsäure (Osteoclasten-Inhibitor, Yamanouchi) Indisulam (p53-Stimulans, Eisai) Aplidin (PPT-Inhibitor, PharmaMar) Rituximab (CD20-Antikörper, Genentech) Gemtuzumab (CD33-Antikörper, Wyeth Ayerst) PG2 (Hämatopoese-Verstärker, Pharmagenesis) Immunol™ (Triclosan-Oralspülung, Endo) Triacetyluridin (Uridin-Prodrug, Wellstat) SN-4071 (Sarkom-Mittel, Signature BioScience) TransMID-107™ (Immunotoxin, KS Biomedix) |
| | PT-100 (Wachstumsfaktor-Agonist, Point Therapeutics) Midostaurin (PKC-Inhibitor, Novartis) Bryostatin-1 (PKC-Stimulans, GPC Biotech) CDA-II (Apoptose-Förderer, Everlife) SDX-101 (Apoptose-Förderer, Salmedix) Ceflatonin (Apoptose-Förderer, ChernGenex) | PCK-3145 (Apoptose-Förderer, Procyon) Doranidazol (Apoptose-Förderer, Pola) CHS-828 (cytotoxisches Mittel, Leo) trans-Retinsäure (Differentiator, NIH) MX6 (Apoptose-Förderer, MAXIA) Apomin (Apoptose-Förderer, ILEX Oncology) Urocidin (Apoptose-Förderer, Bioniche) Ro-31-7453 (Apoptose-Förderer, La Roche) Brostallicin (Apoptose-Förderer, Pharmacia) |
| | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### Test für die Hemmung von IKKε

### IKKε - Kinase-Assay (IKKepsilon)

### Zusammenfassung

Der Kinase-Assay wird als 384-Well-Flashplate-Assay durchgeführt (z.B. für Topcount-Messung).

1 nM IKKε, 800 nM biotinyliertes IκBα(19-42) Peptid (Biotin-C6-C6-GLKKERLLDDRHDSGLDSMKDEE) und 10 µM ATP (gespikt mit 0,3 µCi ³³P-ATP/Well) werden in einem Gesamtvolumen von 50 µl (10 mM MOPS, 10 mM Mg-acetat, 0,1 mM EGTA, 1 mM Dithiothreitol, 0,02 % Brij35, 0,1 % BSA, 0,1 % BioStab, pH 7,5) mit oder ohne Testverbindung für 2 Stunden bei 30°C inkubiert. Die Reaktion wird mit 25 µl 200 mM EDTA gestoppt. Nach 30 Min bei Raumtemperatur wird die Flüssigkeit entfernt und jeder Well dreimal mit 100 µl 0,9%iger Natriumchloridlösung gewaschen. Unspezifische Reaktion wird in Gegenwart von 3 µM MSC2119074 (BX-795) bestimmt. Die Radioaktivität wird mit einem Topcount (PerkinElmer) gemessen. Die Ergebnisse (z.B. IC₅₀-Werte) werden mit durch die IT-Abteilung breitgestellten Programm-Tools (z.B. AssayExplorer, Symyx) berechnet.

### Test für die Hemmung von TBK1

### Enzymtest

### Zusammenfassung

Der Kinase-Assay wird als 384-Well-Flashplate-Assay durchgeführt (z.B. für Topcount-Messung).

0,6 nM TANK Bindungskinase (TBK1), 800 nM biotinyliertes von MELK abgeleitetes Peptid (Biotin-Ah-Ah-AKPKGNKDYHLQTCCGSLAYRRR) und 10 µM ATP (gespikt mit 0,25 µCi ³³P-ATP/Well) werden in einem Gesamtvolumen von 50 µl (10 mM MOPS, 10 mM Mg-acetat, 0,1 mM EGTA, 1 mM DTT, 0,02 % Brij35, 0,1 % BSA, pH 7,5) mit oder ohne Testverbindung 120 Min bei 30°C inkubiert. Die Reaktion wird mit 25 µl 200 mM EDTA gestoppt. Nach 30 Min bei Raumtemperatur wird die Flüssigkeit entfernt und jeder Well dreimal mit 100 µl 0,9%iger Natriumchloridlösung gewaschen. Unspezifische Reaktion wird in Gegenwart von 100 nM Staurosporin gemessen. Die Radioaktivität wird in einem Topcount (PerkinElmer) gemessen. Die Ergebnisse (z.B. IC₅₀-Werte) werden mit durch die IT-Abteilung breitgestellten Programm-Tools (z.B. AssayExplorer, Symyx) berechnet.

### Zelltest

### Dosis-Antwort Hemmung von Phospho-IRF3 @ Ser 386

### cell/MDAMB468/INH/PHOS/IMAG/pIRF3

### 1. Umfang

Obwohl TBK1 und IKKε vor allem als Schlüsselsubstanzen in der angeborenen Immunantwort bekannt sind, weisen neuere Erkenntnisse auf eine Rolle für TBK1 und IKKε in der Ras-induzierten onkogenen Transformation hin. TBK1 wurde als RalB-Effektor im Weg des Ras-like (Ral)-Guanine Nucleotide Exchange Factor (GEF) identifiziert, der für die Ras-induzierte Transformation erforderlich ist. TBK1 aktiviert direkt IRF3, das bei Phosphorylierung homodimerisiert und sich zum Kern verlagert, wo es Prozesse, die mit Entzündung, Immunregulierung, Zellüberleben und Proliferation in Zusammenhang stehen, aktiviert.

Dieser Assay wurde entwickelt, um die Wirksamkeit/Stärke von TBK1/IKKε-Hemmer-Verbindungen auf der Basis der **immunozytochemischen Detektion** von kernlokalisiertem Phospho-IRF3, einem Ziel direkt nach TBK1, zu beurteilen. Behandlung mit Polyinosin-Polycytidylsäure (poly(I:C), einem synthetischen Analogon von doppelsträngiger RNA (dsRNA), ein Molekülmuster, das mit viraler Infektion in Zusammenhang steht und vom Toll-like Rezeptor 3 (TLR3) erkannt wird, wird verwendet, um TBK1/IKKε-Aktivität und IRF3-Phosphorylierung bei Ser386 zu induzieren.

### 2. ASSAY-ÜBERSICHT

1. Tag: MDA-MB-468-Zellen werden mit HyQ-Tase abgelöst, gezählt und auf einer 384-Well-Platte mit TC-Oberfläche und transparentem Boden mit einer Dichte von 10 000 Zellen pro Well in einem Gesamtvolumen von 35 µl Komplettmedium ausgesät. Alternativ werden die Zellen direkt aus tiefgefrorenen Glasfläschchen ausgesät.
2. Tag: Die Zellen werden vor der Poly(I:C)-Stimulierung 1 h mit Hemmer-Verbindungen vorbehandelt. Nach 2h Inkubation mit Poly(I:C) werden die Zellen in (Para)formaldehyd (PFA) fixiert und mit Methanol (MeOH) permeabilisiert. Die Zellen werden dann blockiert und mit einem anti-pIRF3-Antikörper bei 4°C über Nacht inkubiert.
3. Tag: Der primäre Antikörper wird weggewaschen, ein AlexaFluor488-konjugierter sekundärer zugegeben, die Zellen werden mit Propidiumiodid kontrastgefärbt, gefolgt durch Bildaufnahme auf einem IMX Ultra High Content Reader.

### 3. Reagenzien, Materialien

Zellen : ATCC HTB 132, Burger Lab (MP-CB 2010-327 oder MDA-MB-468 / 10)
Plattiermedium = Kulturmedium:
   RPMI 1640, Invitrogen # 31870
   10% FCS, Invitrogen # 10270-106
      2mM Glutamax, Invitrogen #35050-038
   1 mM Natrium-Pyruvat, Invitrogen # 11360
      1 % Pen / Strep
   37°C, 5% CO₂
Platten : 384-Well-Boden-Zellkulturplatten mit schwarzem / transparentem Boden, Falcon #35 3962 oder Greiner #781090
Subkultivierung: HyQ-Tase, Thermo Scientific (HyClone) # SV30030.01
weitere Reagenzien:
   Poly(I:C) (LMW), Invitrogen # tlrl-picw (20mg/ml Stammlösung in steriler PBS herstellen, 30min 55°C im Wasserbad denaturieren, langsam auf RT abkühlen, in Aliquots bei -20°C lagern)
   Referenzhemmer: MSC2119074A-4 = BX-795 (IC50 : 200-800nM)
      Hemmkontrolle: 10µM MSC2119074A-4 = BX-795
      neutrale Kontrolle: 0,5% DMSO
      eine 10Punkt-Dosis-Antwort-Kurve mit MSC2119074A-4 = BX-795 ist in jedem Versuch enthalten
   Hepes, Merck #1.10110
   PBS 1x DPBS , Invitrogen # 14190
   Formaldehyd (methanolfrei, 16%, ultrareine EM-Qualität), Polysciences # 18814 (Lagerung RT), Endkonz.: 4%
   Methanol, Merck # 1.06009.1011 (vorgekühlt -20°C)
   Ziegenserum, PAA # B15-035 (Lagerung 4°C, langfristig -20°C), Endkonz.: 10%
   BSA (IgG- und Protease-frei, 30%), US-Biological # A1317 (Lagerung 4°C, langfristig -20°C), Endkonz.: 2%
      Tween 20 Detergens, Calbiochem # 655204 (Lagerung RT), (10%ige Stammlösung in Wasser herstellen; Endkonz.: 0,1%)
   anti-pIRF-3 Kaninchen MAK, Epitomics # 2526-B (Lagerung -20°C), Endkonz.: 1:2000 in PBS / 2% BSA
      Alexa Fluor Ziege-anti-Kaninchen-488, Invitrogen # A11034 oder # A11008 (Lagerung 4°C, dunkel), Endkonz.: 1:2000 in PBS / 2% BSA / 0,1% Tween
   Propidiumiodid (PI), Fluka # 81845, 1mg/ml in H₂O (Lagerung 4°C, dunkel), Endkonz.: 0,2µg/ml

### 4. Ablauf

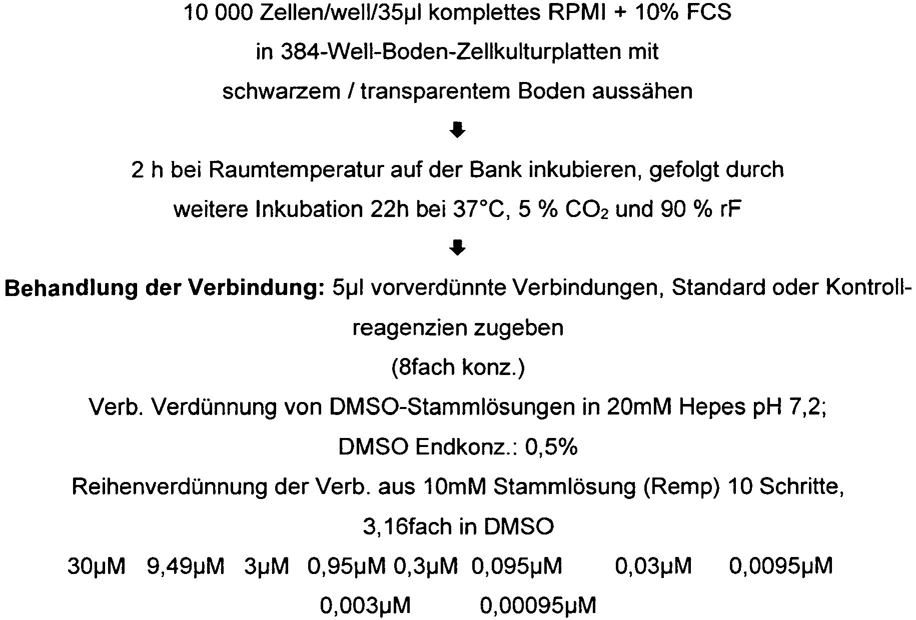

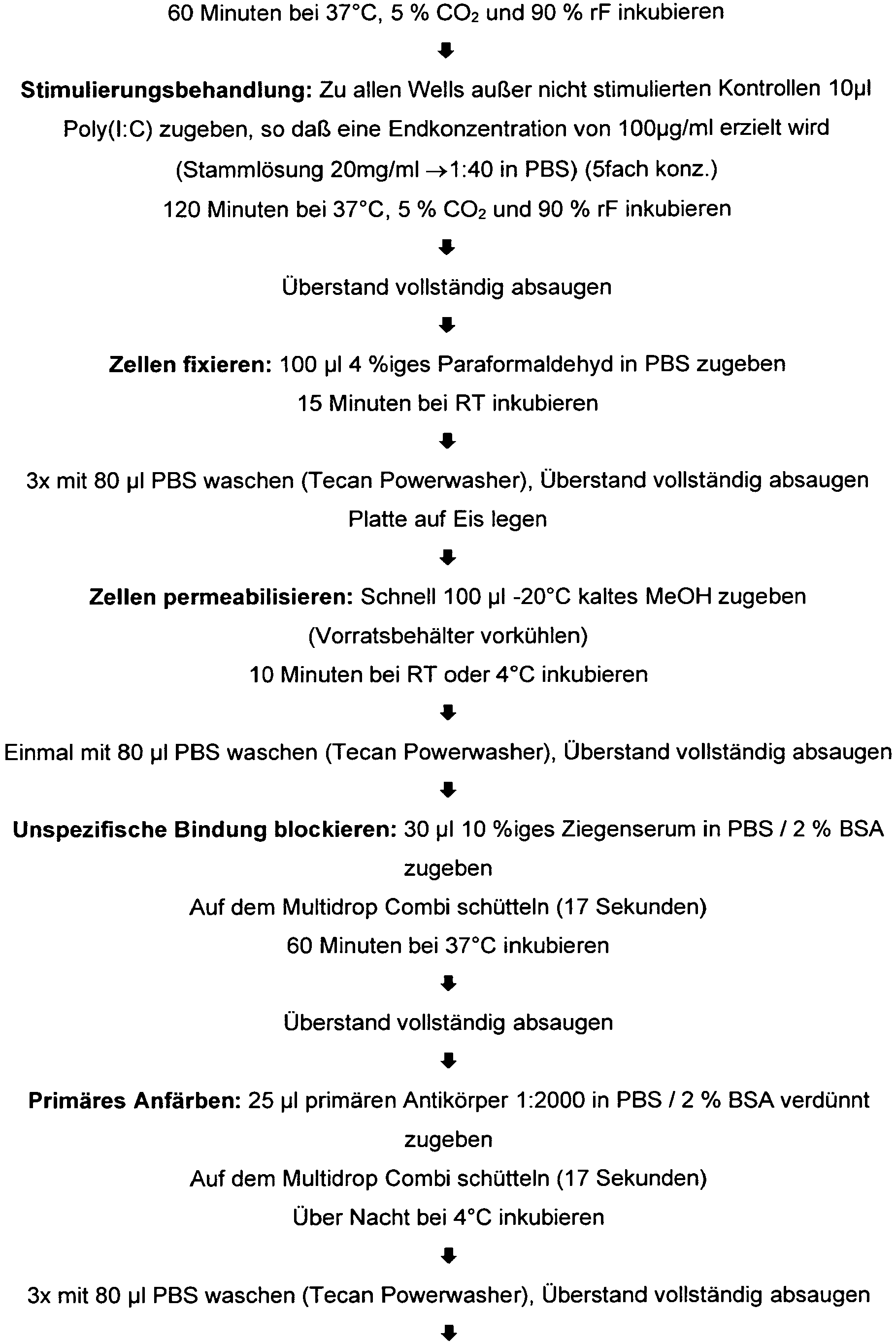

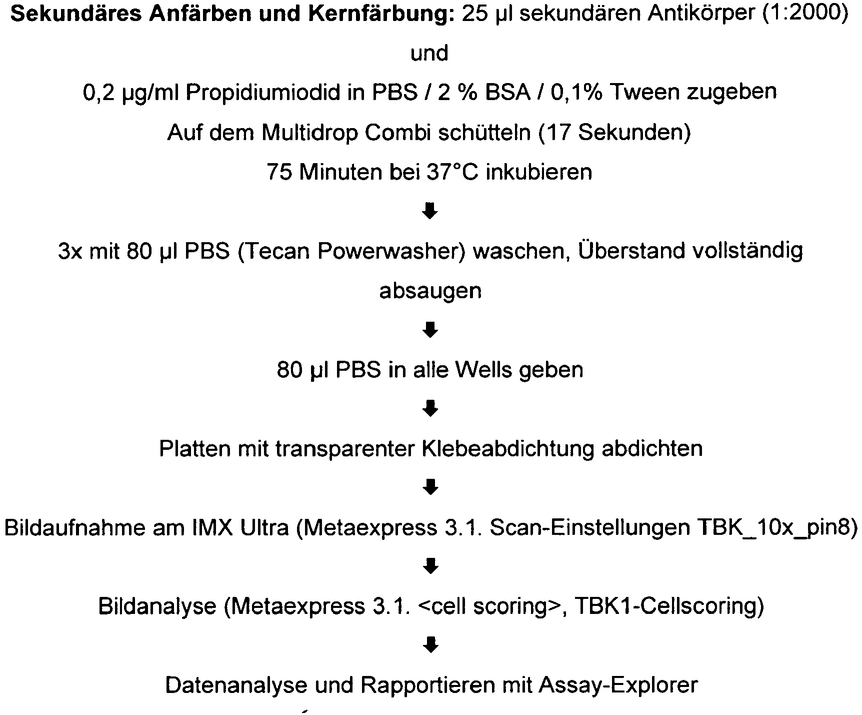

### HPLC/HPLC-MS Bedingungen

Die Retentionszeit Rt [min] wird durch HPLC bestimmt:
Säule: Chromolith SpeedROD RP-18e, 50 x 4.6 mm²
Gradient: A:B = 96:4 to 0:100
Flussrate: 2.4 ml/min
Eluent A: Wasser + 0.05 % Ameisensäure,
Eluent B: Acetonitril + 0.04 % Ameisensäure
Wellenlänge: 220 nm
MS: positive mode

### Beispiele

### Syntheseschema 1

Allgemeiner Syntheseweg für Verbindungen der Formel I, worin X = CH ist. 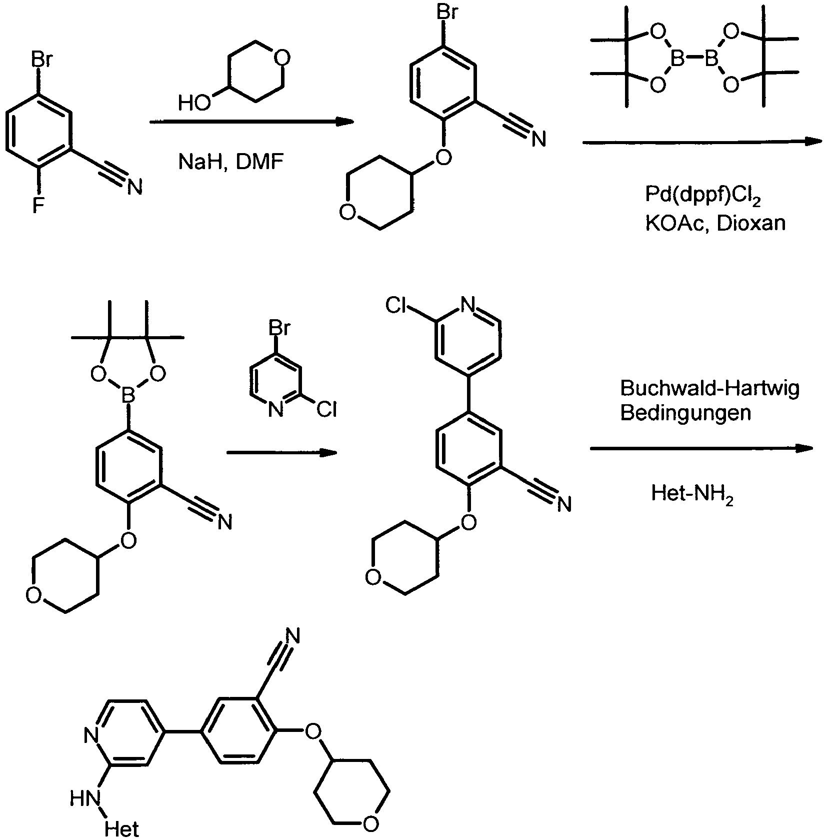 2-(Tetrahydro-pyran-4-yloxy)-5-(4,4,5,5-tetramethyl-[1,3,2]dioxaborolan-2-yl)-benzonitril wird hergestellt wie in WO 2011/046970 A1 beschrieben.

### Synthese von 5-(2-Chlor-pyridin-4-yl)-2-(tetrahydro-pyran-4-yloxy)-benzonitril:

In einem 100 ml Dreihalskolben werden unter N₂ 2-(Tetrahydro-pyran-4-yloxy)-5-(4,4,5,5-tetramethyl-[1,3,2]dioxaborolan-2-yl)-benzonitril (3,645 mmol; 1,20 g) und 4-Brom-2-chlorpyridin (3,645 mmol; 779 mg) in 10 ml Dioxan und 4 mL Wasser gelöst. Es wird mit 1,008 g Kaliumcarbonat und 211mg Tetrakis(triphenylphosphin)-palladium(0) versetzt. Die gelb-braune Lösung wird 2,5 h bei 90°C gerührt.

Zur Aufarbeitung wird das Reaktionsgemisch auf Raumtemperatur gekühlt und mit Wasser und Ethylacetat verdünnt und extrahiert. Die vereinten organischen Phasen werden mit gesättigter NaCl-Lösung gewaschen, getrocknet, filtriert und eingeengt. Man erhält 1,965 g Rohprodukt. Zur Aufreinigung wird das Rohgemisch mit Petrolether/Ethylacetat auf Kieselgel chromatographiert.

Es werden 968 mg des gewünschten Produktes erhalten; HPLC-MS Rt. [min] 2.225; HPLC-MS [M+H] 315;
¹H NMR (500 MHz, DMSO-d₆) δ [ppm]

### Allgemeine Vorschrift für die Buchwald-Hartwig-Reaktion:

In einem 100 ml Dreihalskolben werden unter N₂ 5-(2-Chlor-pyridin-4-yl)-2-(tetrahydro-pyran-4-yloxy)-benzonitril (100 mg; 0,318 mmol), 1.1 Äquivalente der heterozyklischen Aminokomponente, Tris(dibenzylidenaceton)dipalladium(0), 99% (5,8 mg; 0,006 mmol), 9,9-Dimethyl-4,5-bis(diphenylphosphino)xanthen, 99% (36,8 mg; 0,064 mmol), Cäsiumcarbonat (207 mg; 0,635 mmol), und 2-Dicyclohexyl-phosphino-2',4',6'-tri-iso-propyl-1,1'-biphenyl (3,8 mg; 0,008 mmol) in 10 ml Dioxan gelöst. Das Reaktionsgemisch wird dann 4 h auf 140°C erwärmt und über Nacht bei Raumtemperatur gerührt.

Zur Aufarbeitung wird das Lösungsmittel entfernt. Der Rückstand wird mit Wasser verdünnt und mit Dichlormethan extrahiert. Die vereinten organischen Phasen werden mit Wasser gewaschen, getrocknet, filtriert und eingeengt. Der Rückstand wird, wenn nötig, chromatographisch aufgereinigt.

### Herstellung von Verbindungen der Formel I gemäß Anspruch 1 gemäß der allgemeinen Vorschrift für die Buchwald-Hartwig-Reaktion

### 2-(Tetrahydro-pyran-4-yloxy)-5-{2-[1-(3-trifluormethyl-phenyl)-1H-pyrazol-4-ylamino]-pyridin-4-yl}-benzonitril ("A1")

Mit 1-[3-(Trifluormethyl)phenyl]-1H-pyrazol-4-amin wird das gewünschte Produkt in einer Ausbeute von 44% erhalten; HPLC-MS Rt. [min] 2.345; HPLC-MS [M+H] 506;
¹H NMR (500 MHz, DMSO-d₆) δ [ppm] 9.47 (s, 1 H), 9.02 (s, 1 H), 8.35 (d, *J*= 2.4, 1H), 8.23 (m, 2H), 8.14 (dd, *J*= 9.0, 2.4, 1 H), 8.08 (d, *J*= 6.6, 1 H), 8.02 (s, 1 H), 7.81 (t, *J*= 8.3, 1 H), 7.72 (d, J= 7.7, 1 H), 7.56 (d, *J=* 9.1, 1 H), 7.5 - 7.43 (m, 2H), 4.97 (tt, *J*= 7.8, 3.7, 1 H), 3.93 - 3.85 (m, 2H), 3.58 (m, 2H), 2.11 - 2.01 (m, 2H), 1.72 (m, 2H).

### 5-{2-[1-(1-Methyl-piperidin-4-yl)-1H-pyrazol-4-ylamino]-pyridin-4-yl}-2-(tetrahydropyran-4-yloxy)-benzonitril ("A2")

Mit 1-(1-Methyl-piperidin-4-yl)-1H-pyrazol-4-ylamin Hydrochlorid wird das gewünschte Produkt in einer Ausbeute von 6.7% erhalten; HPLC-MS Rt. [min] 1.235; HPLC-MS [M+H] 459;
¹H NMR (500 MHz, DMSO-d₆) δ [ppm] 9.49 (s, 1 H), 8.27 (d, *J*=2.4, 1 H), 8.12 - 8.06 (m, 2H), 8.01 (d, *J*=6.4, 1H), 7.73 (d, *J*=4.0, 1 H), 7.52 (d, *J*=9.2, 1H), 7.41 - 7.37 (m, 2H), 4.96 (m, 1 H), 4.61 - 4.50 (m, 1 H), 3.96 - 3.87 (m, 2H), 3.69 - 3.52 (m, 5H), 3.33 - 3.16 (m, 2H), 2.90 (s, 3H), 2.39 - 2.18 (m, 4H), 2.08 (m, 2H), 1.76 (m, 2H).

### 5-[2-([3,3']Bipyridinyl-6-ylamino)-pyridin-4-yl]-2-(tetrahydro-pyran-4-yloxy)-benzonitril ("A3")

Mit [3,3']Bipyridinyl-6-ylamin wird das gewünschte Produkt in quantitativer Ausbeute erhalten; HPLC-MS Rt. [min] 1.492; HPLC-MS [M+H] 450;
¹H NMR (500 MHz, DMSO-d₆) δ [ppm] 11.36 (s, 1 H), 9.06 (d, *J*=1.9, 1H), 8.77 (d, *J*=2.4, 1 H), 8.70 (dd, *J*=5.0, 1.4, 1 H), 8.41 (d, *J*=6.0, 1 H), 8.38 - 8.30 (m, 2H), 8.25 (d, *J*=2.4, 1 H), 8.10 (dd, *J*=8.9, 2.4, 1 H), 7.79 (d, *J*=0.8, 1 H), 7.71 (dd, *J*=8.0, 5.0, 1 H), 7.64 (d, *J*=8.8, 1 H), 7.61 - 7.52 (m, 2H), 4.96 (m, 1 H), 3.88 (m, 2H), 3.6 (m, 2H), 2.11 - 1.98 (m, 2H), 1.77 - 1.63 (m, 2H).

### 5-[2-(5-Methyl-isoxazol-3-ylamino)-pyridin-4-yl]-2-(tetrahydro-pyran-4-yloxy)-benzonitril ("A4")

Mit 5-Methyl-isoxazol-3-ylamin erhält man das gewünschte Produkt in 30% Ausbeute; HPLC-MS Rt. [min] 1.934; HPLC-MS [M+H] 377;
¹H NMR (500 MHz, DMSO-d₆) δ [ppm] 9.81 (s, 1H), 8.26 (d, *J*=5.3, 1H), 8.08 (d, *J*=2.4, 1 H), 7.96 (dd, *J*=8.9, 2.4, 1 H), 7.64 (m, 1 H), 7.51 (d, *J*=9.1, 1 H), 7.22 (dd, *J*=5.3, 1.6, 1 H), 6.38 (d, *J*=0.6, 1 H), 4.90 (m, 1 H), 3.93 - 3.81 (m, 2H), 3.55 (m, 2H), 2.03 (m, 2H), 1.68 (m, 2H).

### 5-[2-(1-Methyl-1H-pyrazol-3-ylamino)-pyridin-4-yl]-2-(tetrahydro-pyran-4-yloxy)-benzonitril ("A5")

Mit 1-Methyl-1H-pyrazol-3-amin erhält man das gewünschte Produkt in quantitativer Ausbeute; HPLC-MS Rt. [min] 1.558; HPLC-MS [M+H] 376;
¹H NMR (500 MHz, DMSO-d₆) δ [ppm] 11.08 (br, 1H), 8.28 - 8.22 (m, 2H), 8.07 (dd, *J*=9.0, 2.4, 1 H), 7.76 (d, *J*=2.2, 1 H), 7.57 (d, *J*=9.1, 1 H), 7.50 (d, *J*=1.3, 1 H), 7.44 - 7.36 (m, 1 H), 6.20 (d, *J*=2.3, 1 H), 5.00 - 4.88 (m, 1 H), 3.94 - 3.81 (m, 5H), 3.56 (m, 2H), 2.10 - 1.97 (m, 2H), 1.77 - 1.63 (m, 2H).

### 5-[2-(2-Furan-2-ylmethyl-2H-pyrazol-3-ylamino)-pyridin-4-yl]-2-(tetrahydro-pyran-4-yloxy)-benzonitril ("A6")

Mit 2-Furan-2-ylmethyl-2H-pyrazol-3-ylamin erhält man das gewünschte Produkt in 55% Ausbeute; HPLC-MS Rt. [min] 1.908; HPLC-MS [M+H] 442;
¹H NMR (500 MHz, DMSO-d₆) δ [ppm] 8.87 (s, 1H), 8.17 (d, *J*=5.3, 1H), 8.06 (d, *J*=2.4, 1 H), 7.93 (dd, *J*=8.9, 2.4, 1 H), 7.52 (dd, *J*=1.8, 0.8, 1 H), 7.48 (d, *J*=9.1, 1 H), 7.39 (d, *J*=6.9, 1 H), 7.11 (dd, *J*=5.4, 1.6, 1 H), 6.97 (s, 1 H), 5.28 (s, 2H), 4.95 - 4.83 (m, 1 H), 3.94 - 3.83 (m, 2H), 3.59 - 3.51 (m, 2H), 2.08 - 1.95 (m, 2H), 1.72 - 1.60 (m, 2H).

### 5-[2-(5-Morpholin-4-yl-pyridin-2-ylamino)-pyridin-4-yl]-2-(tetrahydro-pyran-4-yloxy)-benzonitril ("A7")

Mit 5-Morpholin-4-yl-pyridin-2-ylamin erhält man das gewünschte Produkt in 23% Ausbeute; HPLC-MS Rt. [min] 1.682; HPLC-MS [M+H] 458;
¹H NMR (500 MHz, DMSO-d₆) δ [ppm] 11.33 (s, 1H), 8.34 (d, *J*=6.3, 1H), 8.24 (t, *J*=7.8, 1 H), 8.08 (dd, *J*=9.0, 2.4, 1 H), 7.95 (d, *J*=2.9, 1 H), 7.81 (d, *J*=7.2, 1 H), 7.58 (d, *J*=9.1, 1H), 7.55 - 7.46 (m, 2H), 7.33 (d, *J*=9.2, 1H), 5.01 - 4.88 (m, 1H), 3.93 - 3.83 (m, 2H), 3.81 - 3.71 (m, 4H), 3.61 - 3.51 (m, 4H), 3.20 - 3.11 (m, 2H), 2.10 - 1.99 (m, 2H), 1.74 - 1.63 (m, 2H).

### 5-[2-(1-Phenyl-1H-pyrazol-4-ylamino)-pyridin-4-yl]-2-(tetrahydro-pyran-4-yloxy)-benzonitril ("A8")

Mit 1-Phenyl-1 H-pyrazol-4-amin erhält man das gewünschte Produkt in 46% Ausbeute; HPLC-MS Rt. [min] 1.977; HPLC-MS [M+H] 438;
¹H NMR (500 MHz, DMSO-d₆) δ [ppm] 9.45 (s, 1H), 8.70 (s, 1H), 8.17 (dd, J=5.5, 4.3, 2H), 8.01 (dd, J=8.9, 2.2, 1 H), 7.85 (s, 1 H), 7.82 (d, J=7.8, 2H), 7.54 - 7.47 (m, 3H), 7.30 (t, J=7.4, 1 H), 7.17 - 7.05 (m, 2H), 4.99 - 4.86 (m, 1 H), 3.93 - 3.80 (m, 2H), 3.60 - 3.49 (m, 2H), 2.11 - 1.97 (m, 2H), 1.75 - 1.61 (m, 2H).

### 5-{2-[5-(1H-Pyrazol-4-yl)-pyridin-2-ylamino]-pyridin-4-yl}-2-(tetrahydro-pyran-4-yloxy)-benzonitril ("A9")

Mit 4-(6-Amino-pyridin-3-yl)-pyrazol-1-carbonsäure-tert.-butylester erhält man das gewünschte Produkt in 16% Ausbeute; HPLC-MS Rt. [min] 1.648; HPLC-MS [M+H] 439.

### 5-[2-(5-tert.-Butyl-1H-pyrazol-3-ylamino)-pyridin-4-yl]-2-(tetrahydro-pyran-4-yloxy)-benzonitril ("A10")

Mit 5-tert.-Butyl-1 H-pyrazol-3-ylamin erhält man das gewünschte Produkt in 8% Ausbeute; HPLC-MS Rt. [min] 1.778; HPLC-MS [M+H] 418;
¹H NMR (500 MHz, DMSO-d₆) δ [ppm] 12.39 (br, 1H), 10.73 (br, 1H), 8.27 (d, *J*=6.3, 1H), 8.22 (s, 1 H), 8.05 (dd, *J*=8.9, 2.3, 1 H), 7.56 (d, *J*=9.0, 2H), 7.34 (s, 1H), 5.96 (s, 1H), 5.00 - 4.88 (m, 1H), 3.95 - 3.80 (m, 2H), 3.61 - 3.53 (m, 2H), 2.10 - 1.97 (m, 2H), 1.77 - 1.62 (m, 2H), 1.31 (s, 9H).

### 6-{4-[3-Cyan-4-(tetrahydro-pyran-4-yloxy)-phenyl]-pyridin-2-ylamino}-nicotinonitril ("A11")

Mit 6-Amino-nicotinonitril erhält man das gewünschte Produkt in 94% Ausbeute; HPLC-MS Rt. [min] 1.738; HPLC-MS [M+H] 398;
¹H NMR (500 MHz, DMSO-d₆) δ [ppm] 10.37 (s, 1H), 8.67 (dd, *J*=2.3, 0.7, 1 H), 8.36 (d, *J*=5.3, 1 H), 8.12 (d, *J*=2.4, 1H), 8.07 (dd, *J*=8.9, 2.3, 1H), 7.99 (dd, *J*=5.9, 3.0, 1H), 7.97 - 7.90 (m, 2H), 7.52 (d, *J*=9.1, 1 H), 7.36 (dd, *J*=5.3, 1.6, 1H), 4.99 - 4.83 (m, 1H), 3.96 - 3.82 (m, 2H), 3.63 - 3.46 (m, 2H), 2.10 - 1.96 (m, 2H), 1.78 - 1.57 (m, 2H).

### 5-[2-(5-Cyclopropyl-2H-pyrazol-3-ylamino)-pyridin-4-yl]-2-(tetrahydro-pyran-4-yloxy)-benzonitril ("A12")

Mit 5-Amino-3-cyclopropyl-1 H-pyrazol erhält man das gewünschte Produkt in 5% Ausbeute; HPLC-MS Rt. [min] 1.674; HPLC-MS [M+H] 402;
¹H NMR (500 MHz, DMSO-d₆) ö [ppm] 10.79 (br, 1H), 8.26 (d, *J*=6.3, 1H), 8.21 (d, *J*=2.0, 1 H), 8.04 (dd, *J*=8.9, 2.4, 1 H), 7.56 (d, *J*=9.1, 1 H), 7.50 (s, 1 H), 7.37 (d, *J*=5.1, 1H), 5.87 (s, 1H), 4.94 (m, 1 H), 3.87 (m, 2H), 3.55 (m, 2H), 2.13 - 1.87 (m, 3H), 1.69 (m, 2H), 1.07 - 0.94 (m, 2H), 0.83 - 0.67 (m, 2H).

### 2-(Tetrahydro-pyran-4-yloxy)-5-[2-(5-trifluormethyl-pyridin-2-ylamino)-pyridin-4-yl]-benzonitril ("A13")

Mit 5-Trifluormethyl-pyridin-2-ylamin erhält man das gewünschte Produkt in 34% Ausbeute; HPLC-MS Rt. [min] 1.917; HPLC-MS [M+H] 441;
¹H NMR (500 MHz, DMSO-d₆) δ [ppm] 10.25 (s, 1H), 8.60 (s, 1 H), 8.34 (d, *J*=5.3, 1 H), 8.13 (d, *J*=2.4, 1H), 8.04 - 7.95 (m, 4H), 7.52 (d, *J*=9.1, 1H), 7.32 (dd, *J*=15.1, 7.5, 1 H), 4.99 - 4.84 (m, 1H), 3.92 - 3.80 (m, 2H), 3.61 - 3.50 (m, 2H), 2.10 - 1.98 (m, 2H), 1.75 - 1.61 (m, 2H).

### 5-[2-(Pyrimidin-2-ylamino)-pyridin-4-yl]-2-(tetrahydro-pyran-4-yloxy)-benzonitril ("A14")

Mit Pyrimidin-2-ylamin erhält man das gewünschte Produkt in 95% Ausbeute; HPLC-MS Rt. [min] 1.508; HPLC-MS [M+H] 374;
¹H NMR (500 MHz, DMSO-d₆) δ [ppm] 9.89 (s, 1H), 8.58 (d, *J*=4.8, 2H), 8.51 (d, *J*=0.8, 1 H), 8.34 (d, *J*=5.2, 1H), 8.13 (d, J=2.4, 1H), 8.01 (dd, *J*=8.9, 2.4, 1H), 7.51 (d, *J*=9.0, 1 H), 7.33 (dd, *J*=5.2, 1.6, 1 H), 6.97 (t, *J*=4.8, 1H), 4.97 - 4.85 (m, 1H), 3.91 - 3.82 (m, 2H), 3.61 - 3.49 (m, 2H), 2.09 - 1.97 (m, 2H), 1.76 - 1.63 (m, 2H).

### 5-[2-(5-Hydroxymethyl-pyridin-2-ylamino)-pyridin-4-yl]-2-(tetrahydro-pyran-4-yloxy)-benzonitril ("A15")

Mit (6-Amino-pyridin-3-yl)-methanol erhält man das gewünschte Produkt in 31% Ausbeute; HPLC-MS Rt. [min] 1.536; HPLC-MS [M+H] 403;
¹H NMR (500 MHz, DMSO-d₆) δ [ppm] 11.41 (br, 1H), 8.42 (d, *J*=6.0, 1H), 8.31 (d, *J*=1.2, 1H), 8.25 (d, *J*=2.3, 1H), 8.08 (dd, *J*=8.9, 2.4, 1H), 8.00 (d, *J*=8.5, 1H), 7.63 - 7.54 (m, 3H), 7.45 (d, *J*=8.6, 1 H), 5.04 - 4.90 (m, 1H), 4.56 (s, 2H), 3.94 - 3.84 (m, 2H), 3.62 - 3.51 (m, 2H), 2.11 - 2.00 (m, 2H), 1.77 - 1.62 (m, 2H).

### 5-[2-(1-Piperidin-4-yl-1H-pyrazol-4-ylamino)-pyridin-4-yl]-2-(tetrahydro-pyran-4-yloxy)-benzonitril ("A16")

Mit 4-(4-Amino-pyrazol-1-yl)-piperidin-1-carbonsäure-tert.-butylester erhält man 4-(4-{4-[3-Cyan-4-(tetrahydro-pyran-4-yloxy)-phenyl]-pyridin-2-ylamino}-pyrazol-1-yl)-piperidin-1-carbonsäure-tert.-butylester in 40% Ausbeute.

87 mg des so erhaltenen tert.-Butylesters werden in 3 mL getrocknetem Dioxan gelöst und mit 3mL 4 molarer HCl in Dioxan versetzt. Die leicht gelbe Lösung läßt man 1 h bei RT rühren.

Die Reaktionslösung wird einrotiert und der pulvrige Rückstand wird mit Petrolether und Ethylacetat verrieben und abgesaugt. Die Substanz wird mehrmals gefriergetrocknet. Man erhält 38,8 mg des gewünschten Produktes; HPLC-MS Rt. [min] 1.244; HPLC-MS [M+H] 445;
¹H NMR (500 MHz, DMSO-d₆) δ [ppm] 8.78 (s, 1 H), 8.15 (d, *J*=5.4, 1H), 8.04 (d, *J*=4.5, 1H), 7.97 (d, 1H), 7.92 (dt, *J*=17.9, 8.9, 1H), 7.51 - 7.43 (m, 2H), 6.93 (dd, *J*=5.4, 1.5, 1H), 6.86 (s, 1H), 4.96 - 4.84 (m, 1 H), 4.22 - 4.08 (m, 1H), 3.95 - 3.82 (m, 2H), 3.59 - 3.47 (m, 2H), 3.10 - 3.02 (m, 2H), 2.61 (td, *J*=12.3, 2.1, 2H), 2.08 - 1.98 (m, 2H), 1.98 - 1.89 (m, 2H), 1.84 - 1.73 (m, 2H), 1.73 - 1.61 (m, 2H).

### 2-{4-[3-Cyan-4-(tetrahydro-pyran-4-yloxy)-phenyl]-pyridin-2-ylamino}-isonicotinonitril ("A 17")

Mit 2-Amino-isonicotinonitril erhält man das gewünschte Produkt in 9% Ausbeute; HPLC-MS Rt. [min] 1.719; HPLC-MS [M+H] 398;
¹H NMR (500 MHz, DMSO-d₆) δ [ppm] 10.34 (s, 1H), 8.49 (d, *J*=5.1, 1H), 8.36 (d, *J*=5.5, 1H), 8.25 (s, 1H), 8.13 (d, *J*=2.3, 1H), 8.00 (dd, *J*=8.9, 2.4, 1H), 7.80 (d, *J*=0.9, 1 H), 7.53 (d, *J*=9.0, 1H), 7.39 - 7.33 (m, 1 H), 7.31 (dd, *J*=5.1, 0.9, 1H), 4.92 (tt, *J*=7.8, 3.8, 1H), 3.91 - 3.82 (m, 2H), 3.56 (ddd, *J*=11.5, 8.4, 3.1, 2H), 2.08 - 1.98 (m, 2H), 1.74 - 1.63 (m, 2H).

### 5-[2-(4-Hydroxymethyl-pyridin-2-ylamino)-pyridin-4-yl]-2-(tetrahydro-pyran-4-yloxy)-benzonitril ("A18")

Mit (2-Amino-pyridin-4-yl)-methanol erhält man das gewünschte Produkt in 60% Ausbeute; HPLC-MS Rt. [min] 1.567; HPLC-MS [M+H] 403;
¹H NMR (500 MHz, DMSO-d₆) δ [ppm] 11.52 (s, 1H), 8.41 (d, J=5.8, 1H), 8.29 (d, *J*=6.0, 1 H), 8.21 (d, *J*=2.3, 1 H), 8.05 (dd, *J*=8.9, 2.4, 1H), 7.55 (d, *J*=9.0, 3H), 7.43 (s, 1 H), 7.13 (d, *J*=5.7, 1 H), 5.56 (br, 1 H), 4.99 - 4.88 (m, 1H), 4.65 (s, 2H), 3.93 - 3.83 (m, 2H), 3.63 - 3.49 (m, 2H), 2.11 - 1.97 (m, 2H), 1.77 - 1.61 (m, 2H).

### 5-{4-[3-Cyan-4-(tetrahydro-pyran-4-yloxy)-phenyl]-pyridin-2-ylamino}-benzofuran-2-carbonsäure-amid ("A19")

Mit 5-Amino-benzofuran-2-carbonsäure-amid erhält man das gewünschte Produkt in 51% Ausbeute; HPLC-MS Rt. [min] 1.824; HPLC-MS [M+H] 455;
¹H NMR (500 MHz, DMSO-d₆) δ [ppm] 10.94 (br, 1H), 9.2 (br, 2H), 8.48 (d, *J*=5.2, 1H), 8.42 (d, *J*=1.0, 1H), 8.19 (d, *J*=2.4, 1H), 8.04 (dd, *J*=8.9, 2.4, 1H), 7.98 (s, 1H), 7.74 (d, *J*=8.8, 1H), 7.59 - 7.50 (m, 3H), 7.30 (dd, *J*=8.8, 1.9, 1H), 4.98 - 4.87 (m, 1 H), 3.92 - 3.83 (m, 2H), 3.62 - 3.50 (m, 2H), 2.09 - 1.98 (m, 2H), 1.75 - 1.63 (m, 2H).

### 2-(Tetrahydro-pyran-4-yloxy)-5-[2-(5,6,7,8-tetrahydro-pyrido[4,3-d]pyrimidin-2-ylamino)-pyridin-4-yl]-benzonitril ("A36")

In einem 50 mL Kolben wird 2-Amino-5,6,7,8-tetrahydropyrido-[4,3-d]pyrimidin dihydrochlorid (100 mg; 0,448 mmol) in 10 mL Dichlormethan gelöst und unter rühren Di-tert.-butyldicarbonat (0,14 ml; 0,672 mmol) und Triethylamin (0,062 ml; 0,448 mmol) zugegeben. Das Reaktionsgemisch wird bei RT über Nacht gerührt. Zur Aufarbeitung wird das Reaktionsgemisch eingeengt. Der Rückstand wird in Essigester verrieben und abgesaugt. Das Filtrat wird eingeengt und man erhält 80 mg 2-Amino-7,8-dihydro-5H-pyrido[4,3-d]pyrimidin-6-carbonsäure-tert.-butylester; HPLC-MS Rt. [min] 1.504; HPLC-MS [M+H] 251;

Mit dem hergestellten 2-Amino-7,8-dihydro-5H-pyrido[4,3-d]pyrimidin-6-carbonsäure-tert.-butylester erhält man nach Buchwald-Hartwig-Bedingungen 2-{4-[3-Cyan-4-(tetrahydro-pyran-4-yloxy)-phenyl]-pyridin-2-ylamino}-7,8-dihydro-5H-pyrido[4,3-d]pyrimidin-6-carbonsäure-tert.-butylester.

2-{4-[3-Cyan-4-(tetrahydro-pyran-4-yloxy)-phenyl]-pyridin-2-ylamino}-7,8-dihydro-5H-pyrido[4,3-d]pyrimidin-6-carbonsäure-tert.-butylester (155 mg; 0,241 mmol) werden in 3,5 mL getrocknetem Dioxan gelöst und mit 3 mL HCl in Dioxan (4 mol/L) versetzt. Die gelbe Lösung wird 30 min bei Raumtemepratur gerührt.

Das Reaktionsgemisch wird mit 2 molarer NaOH basisch gestellt. Der Niederschlag wird abgesaugt und mit Dioxan gewaschen. Man erhält 97 mg vom gewünschten Produkt; HPLC-MS Rt. [min] 1.223; HPLC-MS [M+H] 429;
NMR

### 6-{4-[3-Cyan-4-(tetrahydro-pyran-4-yloxy)-phenyl]-pyridin-2-ylamino}-nicotinamid ("A37")

Mit 6-Amino-nicotinamid erhält man das gewünschte Produkt in 5% Ausbeute; HPLC-MS Rt. [min] 1.476; HPLC-MS [M+H] 416;
¹H NMR (500 MHz, DMSO-d₆) δ [ppm] 10.90 (s, 1 H), 8.68 (d, *J*=1.9, 1H), 8.44 (d, *J*=5.3, 1H), 8.41 (d, *J*=1.0, 1H), 8.28 (dd, *J*=9.1, 2.0, 1H), 8.16 (d, *J*=2.4, 1H), 8.02 (dd, *J*=8.9, 2.4, 1 H), 7.98 - 7.63 (m, 1H), 7.57 - 7.49 (m, 2H), 6.85 (d, *J*=9.1, 1H), 4.98 - 4.87 (m, 1 H), 3.92 - 3.83 (m, 2H), 3.60 - 3.51 (m, 2H), 2.10 - 1.97 (m, 2H), 1.76 - 1.61 (m, 2H).

### Syntheseschema 2

### Herstellung von 1 H-Pyrazol-4-ylaminderivaten

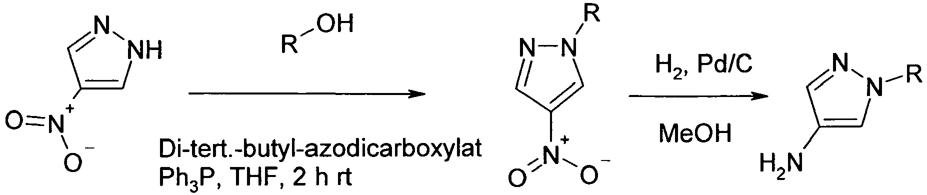

### Allgemeine Vorschrift:

In einem 100 ml Dreihalskolben mit Trockenrohr werden unter N₂, 4-Nitro-1H-pyrazol (4,422 mmol; 500,00 mg), 1 Äquivalent des primären Alkohols und 1,77 g Triphenylphosphin in 20 ml getrocknetem THF gelöst. Anschließend wird portionsweise Di-tert.-butyl-azodicarboxylat (5,748 mmol; 1,35 g) zugegeben. Die gelbe Lösung wird 2 h bei RT gerührt.
Zur Aufarbeitung wird das Triphenylphosphinoxid abgesaugt und das Filtrat einrotiert. Das 4-Nitro-1H-pyrazolderivat, wird, falls erforderlich, über Kieselgel in Ethylacetat/Petrolether chromatographiert.
Das 4-Nitro-1H-pyrazolderivat wird in Methanol gelöst, Pd-C-5% hinzugegeben und mit Wasserstoff bei Raumtemperatur hydriert. Man erhält das 1 H-Pyrazol-4-ylaminderivat nach der Filtration und dem Einengen der Lösung.

1-(2,2-Difluor-ethyl)-1 H-pyrazol-4-ylamin wird hergestellt mit 2,2-Difluorethanol; HPLC-MS Rt. [min] 0.351; HPLC-MS [M+H] 148.

4-[2-(4-Amino-pyrazol-1-yl)-ethyl]-piperidin-1-carbonsäure-tert.-butylester wird hergestellt mit 4-(2-Hydroxy-ethyl)-piperidin-1-carbonsäure-tert.-butylester; HPLC-MS Rt. [min] 1.357; HPLC-MS [M+H] 295.

1-(2-Morpholin-4-yl-ethyl)-1 H-pyrazol-4-ylamin wird hergestellt mit N-(2-Hydroxyethyl)-morpholin; HPLC-MS Rt. [min] 0.320; HPLC-MS [M+H] 197.

1-(3-Methoxy-propyl)-1H-pyrazol-4-ylamin wird hergestellt mit 3-Methoxy-1-propanol; HPLC-MS Rt. [min] 0.363; HPLC-MS [M+H] 155.

2-(4-Amino-pyrazol-1-ylmethyl)-cyclopropancarbonitril wird hergestellt mit 2-Hydroxymethyl-cyclopropancarbonitril; HPLC-MS Rt. [min] 0.380; HPLC-MS [M+H] 163.

3-(4-Amino-pyrazol-1-yl)-azetidin-1-carbonsäure-tert.-butylester wird hergestellt mit 3-Hydroxy-azetidin-1-carbonsäure-tert.-butylester; HPLC-MS Rt. [min] 1.117; HPLC-MS [M+H] 183.

[trans-2-(4-Amino-pyrazol-1-ylmethyl)-cyclopropyl]-methanol wird hergestellt mit trans-2-Hydroxymethyl-cyclopropyl)-methanol; HPLC-MS Rt. [min] 0.355; HPLC-MS [M+H] 168.

1-(Tetrahydro-furan-3-ylmethyl)-1H-pyrazol-4-ylamin wird hergestellt mit (Tetrahydro-furan-3-yl)-methanol; HPLC-MS Rt. [min] 0.357; HPLC-MS [M+H] 168.

3-(4-Amino-pyrazol-1-yl)-pyrrolidin-1-carbonsäure-tert.-butylester wird hergestellt mit 3-Hydroxy-pyrrolidin-1-carbonsäure-tert.-butylester; HPLC-MS Rt. [min] 1.099; HPLC-MS [M+H] 253.

1-(2-Pyrazol-1-yl-ethyl)-1 H-pyrazol-4-ylamin wird hergestellt mit 2-(1H-Pyrazol-1-yl)ethanol; HPLC-MS Rt. [min] 0.355; HPLC-MS [M+H] 178.

### Herstellung von Verbindungen der Formel I gemäß Anspruch 1

### 5-{2-[1-(2,2-Difluor-ethyl)-1H-pyrazol-4-ylamino]-pyridin-4-yl}-2-(tetrahydro-pyran-4-yloxy)-benzonitril ("A20")

Mit dem oben beschriebenen 1-(2,2-Difluor-ethyl)-1 H-pyrazol-4-ylamin erhält man das gewünschte Produkt in 34% Ausbeute; HPLC-MS Rt. [min] 1.619;
HPLC-MS [M+H] 426;
¹H NMR (500 MHz, DMSO-d₆) δ [ppm] 8.94 (s, 1H), 8.16 (d, *J*=8.1, 1H), 8.10 (s, 1H), 8.06 (d, *J*=2.4, 1H), 7.94 (dd, *J*=8.9, 2.4, 1H), 7.55 (s, 1H), 7.47 (d, *J*=10.1, 1H), 6.97 (dd, *J*=5.4, 1.5, 1H), 6.89 (d, *J*=0.7, 1H), 6.33 (tt, *J*=55.1, 3.9, 1 H), 4.95 - 4.83 (m, 1H), 4.66 - 4.50 (m, 2H), 3.93 - 3.82 (m, 2H), 3.62 - 3.48 (m, 2H), 2.08 - 1.96 (m, 2H), 1.74 - 1.60 (m, 2H).

### 5-{2-[1-(2-Piperidin-4-yl-ethyl)-1H-pyrazol-4-ylamino]-pyridin-4-yl}-2-(tetrahydropyran-4-yloxy)-benzonitril ("A21")

Mit dem oben hergestellten 4-[2-(4-Amino-pyrazol-1-yl)-ethyl]-piperidin-1-carbonsäure-tert.-butylester erhält man 4-[2-(4-{4-[3-Cyan-4-(tetrahydro-pyran-4-yloxy)-phenyl]-pyridin-2-ylamino}-pyrazol-1-yl)-ethyl]-piperidin-1-carbonsäure-tert.-butylester in 41 % Ausbeute.

210 mg 4-[2-(4-{4-[3-Cyan-4-(tetrahydro-pyran-4-yloxy)-phenyl]-pyridin-2-ylamino}-pyrazol-1-yl)-ethyl]-piperidin-1-carbonsäure-tert.-butylester werden in 5 mL getrocknetem Dioxan gelöst und mit 5 mL HCl in Dioxan (4 mol/L) versetzt. Die gelbe Lösung wird 30 min bei Raumtemperatur gerührt.

Das Reaktionsgemisch wird mit 2 molarer NaOH basisch gestellt und extrahiert. Die vereinten organischen Phasen werden getrocknet, filtriert und eingeengt. Man erhält 150 mg der gewünschten Verbindung; HPLC-MS Rt. [min] 1.274;
HPLC-MS [M+H] 473;
¹H NMR (500 MHz, DMSO-d₆) δ [ppm] 8.78 (d, 1H), 8.15 (d, *J*=5.4, 1 H), 8.04 (d, *J*=2.4, 1H), 7.97 (s, 1H), 7.92 (dd, *J*=8.9, 2.4, 1 H), 7.47 (d, *J*=9.1, 1 H), 7.44 (s, 1H), 6.93 (dd, *J*=5.4, 1.6, 1H), 6.86 (d, *J*=0.8, 1 H), 4.96 - 4.82 (m, 1H), 4.15 - 4.04 (m, 2H), 3.91 - 3.81 (m, 2H), 3.59 - 3.51 (m, 2H), 2.99 - 2.85 (m, 2H), 2.47 - 2.36 (m, 2H), 2.10 - 1.96 (m, 2H), 1.74 - 1.52 (m, 6H), 1.34 - 0.98 (m, 3H).

### 5-{2-[1-(2-Morpholin-4-yl-ethyl)-1H-pyrazol-4-ylamino]-pyridin-4-yl}-2-(tetrahydropyran-4-yloxy)-benzonitril ("A22")

Mit dem oben hergestellten 1-(2-Morpholin-4-yl-ethyl)-1 H-pyrazol-4-ylamin erhält man das gewünschte Produkt in 42% Ausbeute; HPLC-MS Rt. [min] 1.307;
HPLC-MS [M+H] 475;
¹H NMR (500 MHz, DMSO-d₆) δ [ppm] 9.03 (br, 1 H), 8.20 - 8.13 (m, 2H), 8.08 (d, *J*=2.3, 1 H), 7.96 (dd, *J*=8.9, 2.4, 1 H), 7.58 (s, 1H), 7.50 (d, *J*=9.1, 1 H), 7.01 (d, *J*=5.0, 1 H), 6.93 (s, 1H), 4.96 - 4.85 (m, 1H), 4.53 (t, *J*=6.1, 2H), 3.96 - 3.83 (m, 6H), 3.61 - 3.52 (m, 8H), 2.08 - 1.97 (m, 2H), 1.75 - 1.57 (m, 2H).

### 5-{2-[1-(3-Methoxy-propyl)-1H-pyrazol-4-ylamino]-pyridin-4-yl}-2-(tetrahydro-pyran-4-yloxy)-benzonitril ("A23")

Mit dem oben hergestellten 1-(3-Methoxy-propyl)-1H-pyrazol-4-ylamin erhält man das gewünschte Produkt in 16% Ausbeute; HPLC-MS Rt. [min] 1.565;
HPLC-MS [M+H] 434;
¹H NMR (500 MHz, DMSO-d₆) δ [ppm] 9.41 (br, 1H), 8.17 (d, J=2.0, 1 H), 8.06 (d, *J*=6.0, 1 H), 8.02 - 7.97 (m, 2H), 7.55 (s, 1 H), 7.51 (d, *J*=9.1, 1 H), 7.16 (s, 1 H), 7.07 (s, 1 H), 5.01 - 4.84 (m, 1 H), 4.14 (t, J=7.0, 2H), 3.91 - 3.78 (m, 3H), 3.32 (t, *J*=6.2, 2H), 3.24 (s, 3H), 2.11 - 1.95 (m, 4H), 1.76 - 1.58 (m, 2H).

### 5-{2-[1-(2-Cyan-cyclopropylmethyl)-1H-pyrazol-4-ylamino]-pyridin-4-yl}-2-(tetrahydro-pyran-4-yloxy)-benzonitril ("A24")

Mit dem oben hergestellten 2-(4-Amino-pyrazol-1-ylmethyl)-cyclopropancarbonitril erhält man das gewünschte Produkt in 28% Ausbeute; HPLC-MS Rt. [min] 1.573;
HPLC-MS [M+H] 431;
¹H NMR (500 MHz, DMSO-d₆) δ [ppm] 9.49 (br, 1H), 8.17 (d, *J*=2.2, 1H), 8.08 (d, *J*=6.0, 1 H), 8.06 (s, 1H), 8.00 (dd, *J*=8.9, 2.4, 1H), 7.59 (s, 1H), 7.51 (d, *J*=9.1, 1H), 7.15 (d, *J*=5.5, 1H), 7.09 (s, 1H), 4.98 - 4.86 (m, 1H), 4.18 - 4.10 (m, 1H), 4.10 - 4.00 (m, 1H), 3.92 - 3.82 (m, 2H), 3.60 - 3.49 (m, 2H), 2.07 - 1.90 (m, 3H), 1.86 - 1.78 (m, 1H), 1.74 - 1.63 (m, 2H), 1.35 - 1.27 (m, 1H), 1.17 - 1.09 (m, 1 H).

### 5-[2-(1-Azetidin-3-yl-1H-pyrazol-4-ylamino)-pyridin-4-yl]-2-(tetrahydro-pyran-4-yloxy)-benzonitril ("A25")

Mit dem oben hergestellten 3-(4-Amino-pyrazol-1-yl)-azetidin-1-carbonsäure-tert.-butylester erhält man 3-(4-{4-[3-Cyan-4-(tetrahydro-pyran-4-yloxy)-phenyl]-pyridin-2-ylamino}-pyrazol-1-yl)-azetidin-1-carbonsäure-tert.-butylester in 18% Ausbeute. 71 mg 3-(4-{4-[3-Cyan-4-(tetrahydro-pyran-4-yloxy)-phenyl]-pyridin-2-ylamino}-pyrazol-1-yl)-azetidin-1-carbonsäure-tert.-butylester werden in 3 mL Dioxan gelöst und mit 3 mL HCl in Dioxan (4 molar) versetzt. Die gelbe Lösung wird 30 min bei Raumtemperatur gerührt.

Zur Aufarbeitung wird die Reaktionslösung mit 2 molarer NaOH basisch gestellt und mit Ethylacetat extrahiert. Die vereinten organischen Phasen werden getrocknet, filtriert und eingeengt. Nach Chromatographie auf Kieselgel erhält man 27 mg der gewünschten Verbindung; HPLC-MS Rt. [min] 1.255; HPLC-MS [M+H] 417.

### 5-{2-[1-((1S,2S)-2-Hydroxymethyl-cyclopropylmethyl)-1H-pyrazol-4-ylamino]-pyridin-4-yl}-2-(tetrahydro-pyran-4-yloxy)-benzonitril ("A26")

Mit dem oben hergestellten [trans-2-(4-Amino-pyrazol-1-ylmethyl)-cyclopropyl]-methanol erhält man das gewünschte Produkt in 35% Ausbeute; HPLC-MS Rt. [min] 1.490; HPLC-MS [M+H] 446;
¹H NMR (500 MHz, DMSO-d₆) δ [ppm] 9.45 (s, 1 H), 8.18 (s, 1 H), 8.11 - 7.95 (m, 2H), 7.58 - 7.48 (m, 2H), 7.16 (s, 1 H), 7.09 (s, 1 H), 4.99 - 4.86 (m, 1 H), 4.08 - 3.93 (m, 2H), 3.87 (dt, *J*=10.3, 3.5, 2H), 3.61 - 3.48 (m, 2H), 3.35 (dd, *J*=11.2, 6.1, 1 H), 3.26 (dd, *J=11.2,* 6.5, 1 H), 2.07 - 1.96 (m, 2H), 1.73 - 1.62 (m, 2H), 1.19 - 0.99 (m, 2H), 0.59 - 0.38 (m, 2H).

### 5-{2-[1-(Tetrahydro-furan-3-ylmethyl)-1H-pyrazol-4-ylamino]-pyridin-4-yl}-2-(tetrahydro-pyran-4-yloxy)-benzonitril ("A27")

Mit dem oben hergestellten 1-(Tetrahydro-furan-3-ylmethyl)-1H-pyrazol-4-ylamin erhält man das gewünschte Produkt in 37% Ausbeute; HPLC-MS Rt. [min] 1.536;
HPLC-MS [M+H] 446;
¹H NMR (500 MHz, DMSO-d₆) δ [ppm] 9.51 (s, 1H), 8.18 (d, *J*=1.6, 1H), 8.09 - 8.03 (m, 2H), 8.01 (dd, *J*=8.9, 2.3, 1 H), 7.57 (s, 1H), 7.52 (d, *J*=9.1, 1 H), 7.18 (d, J=4.2, 1H), 7.10 (s, 1H), 4.98 - 4.87 (m, 1H), 4.17 - 4.04 (m, 2H), 3.90 - 3.83 (m, 2H), 3.77 (td, *J*=8.1, 5.7, 1H), 3.71 - 3.60 (m, 2H), 3.60 - 3.45 (m, 3H), 2.79 - 2.67 (m, 1H), 2.08 - 1.99 (m, 2H), 1.99 - 1.86 (m, 1H), 1.74 - 1.53 (m, 3H).

### 5-[2-(1-Pyrrolidin-3-yl-1H-pyrazol-4-ylamino)-pyridin-4-yl]-2-(tetrahydro-pyran-4-yloxy)-benzonitril ("A28")

Mit dem oben hergestellten 3-(4-Amino-pyrazol-1-yl)-pyrrolidin-1-carbonsäure-tert.-butylester erhält man 3-(4-{4-[3-Cyan-4-(tetrahydro-pyran-4-yloxy)-phenyl]-pyridin-2-ylamino}-pyrazol-1-yl)-pyrrolidin-1-carbonsäure-tert.-butylester in 68% Ausbeute. 110 mg 3-(4-{4-[3-Cyan-4-(tetrahydro-pyra n-4-yloxy)-phenyl]-pyridin-2-ylamino}-pyrazol-1-yl)-pyrrolidin-1-carbonsäure-tert.-butylester werden in 3 mL getrocknetem Dioxan gelöst und mit 3 mL HCl in Dioxan (4 mol/L) versetzt. Die gelbe Lösung wird 30 min bei Raumtemperatur gerührt.

Zur Aufarbeitung wird das Reaktionsgemisch mit 2 molarer NaOH basisch gestellt. Die Lösung wird einrotiert und chromatographiert. Man erhält 100 mg des gewünschten Produkts; HPLC-MS Rt. [min] 1.288; HPLC-MS [M+H] 431;
¹H NMR (500 MHz, DMSO-d₆) δ [ppm] 8.93 (s, 1H), 8.17 (d, *J*=5.4, 1H), 8.11 (s, 1H), 8.07 (d, *J*=2.4, 1H), 7.95 (dd, *J*=8.9, 2.4, 1H), 7.55 (s, 1H), 7.50 (d, *J*=9.1, 1H), 6.97 (dd, *J*=5.4, 1.5, 1H), 6.91 (s, 1H), 5.09 - 5.00 (m, 1H), 4.95 - 4.86 (m, 1H), 3.93 - 3.82 (m, 2H), 3.60 - 3.52 (m, 2H), 3.51 - 3.43 (m, 2H), 3.22 - 3.12 (m, 2H), 2.35 - 2.27 (m, 1H), 2.22 - 2.13 (m, 1H), 2.08 - 1.99 (m, 2H), 1.74 - 1.63 (m, 2H).

### 5-{2-[1-(2-Pyrazol-1-yl-ethyl)-1H-pyrazol-4-ylamino]-pyridin-4-yl}-2-(tetrahydropyran-4-yloxy)-benzonitril ("A38")

Mit dem oben hergestellten 1-(2-Pyrazol-1-yl-ethyl)-1 H-pyrazol-4-ylamin erhält man das gewünschte Produkt in 46% Ausbeute; HPLC-MS Rt. [min] 1.538;
HPLC-MS [M+H] 456;
¹H NMR (500 MHz, DMSO-d₆) δ [ppm] 9.43 (s, 1H), 8.17 (d, *J*=1.7, 1H), 8.07 (d, *J*=6.1, 1 H), 8.00 (dd, *J*=8.9, 2.3, 1 H), 7.75 (s, 1 H), 7.59 (s, 1 H), 7.55 - 7.51 (m, 2H), 7.45 (d, *J*=1.5, 1 H), 7.17 (s, 1 H), 7.00 (s, 1 H), 6.16 (t, *J*=2.0, 1 H), 4.99 - 4.89 (m, 1 H), 4.61 - 4.48 (m, 4H), 3.91 - 3.82 (m, 2H), 3.62 - 3.51 (m, 2H), 2.10 - 1.99 (m, 2H), 1.73 - 1.62 (m, 2H).

### 5-[2-(1-{2-[1-(2-Hydroxy-acetyl)-piperidin-4-yl]-ethyl)-1H-pyrazol-4-ylamino)-pyridin-4-yl]-2-(tetrahydro-pyran-4-yloxy)-benzonitril ("A29")

In einem 50 mL Kolben werden 5-{2-[1-(2-Piperidin-4-yl-ethyl)-1H-pyrazol-4-ylamino]-pyridin-4-yl}-2-(tetrahydro-pyran-4-yloxy)-benzonitril (0,060 mmol; 30,00 mg) und Glycolsäure (0,072 mmol; 5,50 mg) in 5 mL DMF gelöst und mit HATU (0,090 mmol; 34,40 mg) und 4-Methylmorpholin (0,181 mmol; 0,02 ml) versetzt. Die beige Lösung wird 4,5 h bei Raumtemperatur gerührt.

Zur Aufarbeitung wird das DMF abrotiert und der Rückstand mit Ethylacetat und 2 molarer NaOH extrahiert. Die organischen Phasen werden getrocknet, filtriert und eingeengt.

Das erhaltene Rohprodukt wird über Kieselgel chromatographiert (Dichlormethan, Methanol).

Man erhält 32 mg vom gewünschten Produkt; HPLC-MS Rt. [min] 1.527
HPLC-MS [M+H] 531;
¹H NMR (500 MHz, DMSO-d₆) δ [ppm] 11,94 (br, 1H), 8.79 (s, 1H), 8.15 (d, *J*=5.4, 1 H), 8.05 (d, *J*=2.4, 1 H), 7.99 (s, 1H), 7.92 (dd, *J*=8.9, 2.4, 1 H), 7.51 - 7.40 (m, 1H), 6.94 (dd, *J*=5.4, 1.3, 1 H), 6.86 (s, 1 H), 4.94 - 4.84 (m, 1H), 4.40 (s, 1H), 4.30 (d, *J*=12.6, 1 H), 4.11 (t, *J*=7.1, 2H), 4.07 - 3.99 (m, 2H), 3.91 - 3.82 (m, 2H), 3.66 - 3.58 (m, 1H), 3.58 - 3.49 (m, 2H), 2.87 (t, *J*=12.3, 1H), 2.59 - 2.50 (m, 1H), 2.10 - 1.97 (m, 2H), 1.78 - 1.61 (m, 5H), 1.51 - 1.37 (m, 1 H), 1.16 - 0.91 (m, 3H).

### 5-[2-(1-{2-[1-(2-Amino-acetyl)-piperidin-4-yl]-ethyl}-1H-pyrazol-4-ylamino)-pyridin-4-yl]-2-(tetrahydro-pyran-4-yloxy)-benzonitril ("A30")

In einem 50 mL Kolben werden 5-{2-[1-(2-Piperidin-4-yl-ethyl)-1H-pyrazol-4-ylamino]-pyridin-4-yl}-2-(tetrahydro-pyran-4-yloxy)-benzonitrit (0,121 mmol; 60,00 mg) und BOC-Glycin (0,145 mmol; 25,36 mg) in 10 mL DMF gelöst, mit HATU (0,181 mmol; 68,79 mg) und 4-Methylmorpholin (0,362 mmol; 0,04 ml; 3,00 äq.) versetzt. Die hellgelbe Lösung wird 2 h bei Raumtemperatur gerührt.

Zur Aufarbeitung wird das DMF einrotiert und Rückstand mit Ethylacetat und 2 molarer NaOH extrahiert. Die vereinten organischen Phasen werden getrocknet, filtriert und eingeengt.

Man erhält 127 mg gelbes Öl von (2-{4-[2-(4-{4-[3-Cyan-4-(tetrahydro-pyran-4-yloxy)-phenyl]-pyridin-2-ylamino}-pyrazol-1-yl)-ethyl]-piperidin-1-yl}-2-oxo-ethyl)-carbaminsäure-tert.-butylester.

Diese werden in 5 mL Dioxan gelöst und mit 3 mL HCl in Dioxan (4 molar) versetzt. Die gelbe Lösung wird 1 h bei Raumtemperatur gerührt.

Zur Aufarbeitung wird die Reaktionslösung mit 2 molarer NaOH basisch gestellt, mit Ethylacetat verdünnt und extrahiert. Die vereinten organischen Phasen werden getrocknet, filtriert und eingeengt.

Das erhaltene Rohprodukt wird chromatographisch gereinigt (Kieselgel, Dichlormethan/Methanol). Man erhält 35 mg des gewünschten Produktes; HPLC-MS Rt. [min] 1.323; HPLC-MS [M+H] 530;
¹H NMR (500 MHz, DMSO-d₆) δ [ppm] 8.79 (d, 1 H), 8.14 (d, *J*=6.0, 1H), 8.04 (d, *J*=2.3, 1 H), 7.99 (s, 1 H), 7.92 (dd, *J*=8.9, 2.3, 1 H), 7.47 (d, *J*=6.3, 1H), 7.46 (s, 1H), 6.94 (dd, *J*=5.4, 1.4, 1 H), 6.86 (s, 1H), 4.95 - 4.84 (m, 1H), 4.31 (s, 1H), 4.11 (t, *J*=7.1, 2H), 3.92 - 3.82 (m, 2H), 3.67 (d, *J*=12.4, 1 H), 3.60 - 3.43 (m, 4H), 2.98 - 2.82 (m, 1 H), 2.59 - 2.52 (m, 1 H), 2.09 - 1.98 (m, 2H), 1.79 - 1.61 (m, 6H), 1.54 - 1.35 (m, 1 H), 1.17 - 0.93 (m, 3H).

### Synthese unter Verwendung von Kalium-tert.-butylat

### 5-[2-(3-tert.-Butyl-isoxazol-5-ylamino)-pyridin-4-yl]-2-(tetrahydro-pyran-4-yloxy)-benzonitril ("A31")

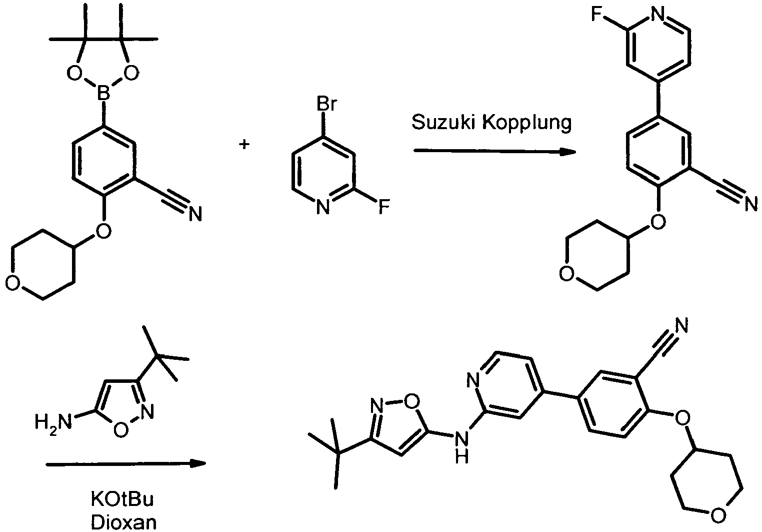

In einem 100 ml Dreihalskolben werden unter N₂ 2-(Tetrahydro-pyran-4-yloxy)-5-(4,4,5,5-tetramethyl-[1,3,2]dioxaborolan-2-yl)-benzonitril (6,766 mmol; 2,75 g) und 4-Brom-2-fluor-pyridin (6,766 mmol; 0,77 ml) in 25 ml Dioxan und 10 mL Wasser gelöst und mit 1,87g Kaliumcarbonat und 392mg Tetrakis(triphenylphosphin)-palladium(0) versetzt. Die dunkelbraune Lösung wird 2,5 h bei 90°C gerührt.

Zur Aufarbeitung wird das Reaktionsgemisch auf Raumtemperatur abgekühlt und mit Wasser und Ethylacetat verdünnt und extrahiert. Die vereinten organischen Phasen werden mit gesättigter NaCl-Lösung gewaschen, getrocknet, filtriert und eingeengt.

Man erhält 3,5 g Rohprodukt, das zur Aufreinigung über Kieselgel chromatographiert wird (Ethylacetat/Petrolether).

Es werden 2,1 g 5-(2-Fluor-pyridin-4-yl)-2-(tetrahydro-pyran-4-yloxy)-benzonitril erhalten; HPLC-MS Rt. [min] 2.135; HPLC-MS [M+H] 299;

In einem 50 mL Dreihalskolben werden 100 mg 5-(2-Fluor-pyridin-4-yl)-2-(tetrahydro-pyran-4-yloxy)-benzonitril unter N₂ in 6 ml Dioxan suspendiert, 52 mg 3-tert-Butyl-isoxazol-5-ylamin und 79 mg KOtBu zugegeben Die gelbe Lösung wird 2,5 h bei 80°C gerührt. Zur Aufarbeitung wird das Reaktionsgemisch einrotiert, der Rückstand wird in Ethylacetat und Wasser aufgenommen und extrahiert. Die gesammelten organischen Phasen werden getrocknet, filtriert und eingeengt.

Das Rohprodukt wird durch präparative HPLC gereinigt. Man erhält das gewünschte Produkt in 46% Ausbeute; HPLC-MS Rt. [min] 2.556; HPLC-MS [M+H] 419;
¹H NMR (500 MHz, DMSO-d₆) δ [ppm] 8.36 (d, *J*=5.7, 1H), 8.19 (d, *J*=2.4, 1H), 8.04 (dd, *J*=8.9, 2.4, 1 H), 7.53 (d, *J*=9.1, 1 H), 7.42 (dd, *J*=5.8, 1.6, 1 H), 7.38 (s, 1 H), 5.00 - 4.88 (m, 1 H), 3.96 - 3.85 (m, 2H), 3.64 - 3.50 (m, 2H), 2.12 - 2.00 (m, 2H), 1.79 - 1.66 (m, 2H), 1.38 - 1.24 (s, 9H).

### Synthese von 5-{2-[5-(1H-Pyrazol-4-yl)-pyridin-2-ylamino]-pyridin-4-yl}-2-(tetrahydro-pyran-4-yloxy)-benzonitril ("A9")

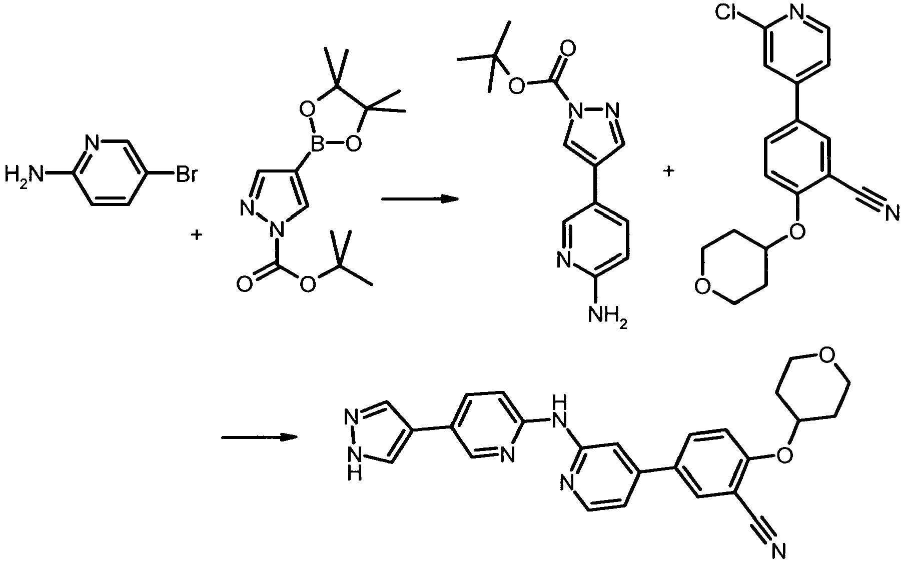

In einem 50 ml Dreihalskolben werden unter N₂ 5-Brom-pyridin-2-ylamin (200 mg; 1,156 mmol) und 4-(4,4,5,5-Tetramethyl-[1,3,2]dioxaborolan-2-yl)-pyrazol-1-carbonsäure-tert.-butylester (420,670 mg; 1,387 mmol) in 3 ml Dioxan und 1 ml Wasser gelöst und mit Kaliumcarbonat (0,131 ml; 2,312 mmol) und Tetrakis(triphenylphosphin)-palladium(0) (133,5 mg; 0,116 mmol) versetzt. Die Lösung wird über Nacht bei 90°C gerührt.

Zur Aufarbeitung wird das Reaktionsgemisch auf Raumtemperatur gekühlt, mit Wasser verdünnt und mit Essigester extrahiert. Die vereinten organischen Phasen werden mit Natriumsulfat getrocknet, filtriert und das Lösungsmittel wird einrotiert. Der Rückstand wird chromatographisch (Kieselgel Dichlormethan/Methanol) gereinigt. Man erhält 249 mg 4-(6-Amino-pyridin-3-yl)-pyrazol-1-carbonsäure-tert.-butylester; HPLC-MS Rt. [min] 1.304; HPLC-MS [M+H] 261. 85 mg 4-(6-Amino-pyridin-3-yl)-pyrazol-1-carbonsäure-tert.-butylester werden mit 100 mg 5-(2-Chlor-pyridin-4-yl)-2-(tetrahydro-pyran-4-yloxy)-benzonitril nach der oben angegebenen allgemeinen Vorschrift für die Buchwald-Hartwig-Reaktion umgesetzt. Man erhält das gewünschte Produkt in 16% Ausbeute;
HPLC-MS Rt. [min] 1.648; HPLC-MS [M+H] 439;
¹H NMR (500 MHz, DMSO-d₆) δ [ppm] 9.69 (s, 1H), 8.54 (d, *J*=2.0, 1H), 8.28 (d, *J*=5.2, 1 H), 8.08 (d, *J*=10.4, 1H), 8.00 - 7.95 (m, 2H), 7.90 (dd, *J*=8.7, 2.4, 2H), 7.80 (d, *J*=8.7, 1H), 7.52 (d, *J*=9.1, 1H), 7.20 (dd, *J*=5.3, 1.6, 1H), 4.96 - 4.83 (m, 1H), 3.92 - 3.83 (m, 2H), 3.60 - 3.52 (m, 2H), 2.08 - 1.98 (m, 2H), 1.76 - 1.64 (m, 2H).

### Syntheseschema 2

Allgemeiner Syntheseweg für Verbindungen der Formel I, worin X = N ist.

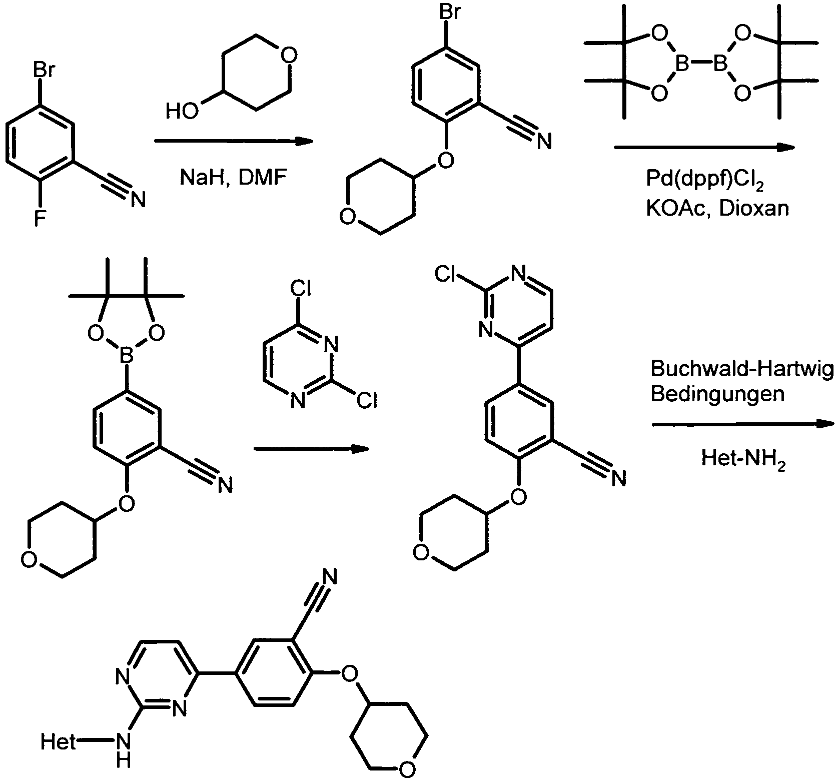

5-(2-Chlor-pyrimidin-4-yl)-2-(tetrahydro-pyran-4-yloxy)-benzonitril wird hergestellt wie in WO 2011/046970 A1 beschrieben.

### Herstellung von Verbindungen der Formel I gemäß Anspruch 1 nach Buchwald-Hartwig

### 2-(Tetrahydro-pyran-4-yloxy)-5-{2-[1-(3-trifluormethyl-phenyl)-1H-pyrazol-4-ylamino]-pyrimidin-4-yl}-benzonitril ("A32")

Mit 1-[3-(Trifluormethyl)phenyl]-1H-pyrazol-4-amin wird das gewünschte Produkt in einer Ausbeute von 12% erhalten; HPLC-MS Rt. [min] 2.717; HPLC-MS [M+H] 507;
¹H NMR (500 MHz, DMSO-d₆) δ [ppm] 9.80 (s, 1 H), 8.75 (s, 1 H), 8.60 - 8.54 (m, 2H), 8.45 (dd, *J*=9.0, 2.2, 1 H), 8.17 - 8.10 (m, 2H), 7.98 (s, 1 H), 7.74 (t, *J*=7.9, 1 H), 7.64 (d, *J*=7.8, 1 H), 7.53 (d, *J*=9.1, 1 H), 7.43 (d, *J*=5.2, 1 H), 5.00 - 4.89 (m, 1 H), 3.92 - 3.83 (m, 2H), 3.60 - 3.51 (m, 2H), 2.10 - 1.99 (m, 2H), 1.75 - 1.63 (m, 2H).

### 5-[2-(1-Methyl-1H-pyrazol-3-ylamino)-pyrimidin-4-yl]-2-(tetrahydro-pyran-4-yloxy)-benzonitril ("A33")

Mit 1-Methyl-1H-pyrazol-3-amin erhält man das gewünschte Produkt in 36% Ausbeute; HPLC-MS Rt. [min] 1.956; HPLC-MS [M+H] 377;
¹H NMR (500 MHz, DMSO-d₆) δ [ppm] 9.70 (s, 1H), 8.49 (dd, *J*=5.8, 3.7, 2H), 8.42 (dd, *J*=9.0, 2.3, 1 H), 7.58 (d, *J*=2.2, 1 H), 7.53 (d, *J*=9.1, 1 H), 7.39 (d, *J*=5.2, 1 H), 6.62 (d, *J*=2.2, 1H), 5.01 - 4.84 (m, 1 H), 3.95 - 3.81 (m, 2H), 3.76 (s, 3H), 3.62 - 3.49 (m, 2H), 2.13 - 1.98 (m, 2H), 1.78 - 1.59 (m, 2H).

### 5-[2-(1H-Pyrazol-4-ylamino)-pyrimidin-4-yl]-2-(tetrahydro-pyran-4-yloxy)-benzonitril ("A34")

Mit 4-Amino-pyrazol-1-carbonsäure-tert.-butylester erhält man das gewünschte Produkt in 4% Ausbeute; HPLC-MS Rt. [min] 1.804; HPLC-MS [M+H] 363;
¹H NMR (500 MHz, DMSO-d₆) δ [ppm] 9.47 (s, 1H), 8.48 (m, 2H), 8.41 (dd, *J*=9.0, 2.2, 1H), 7.79 (s, 2H), 7.53 (d, *J*=9.1, 1H), 7.32 (d, *J*=5.2, 1H), 4.97 - 4.87 (m, 1H), 3.92 - 3.82 (m, 2H), 3.60 - 3.49 (m, 2H), 2.10 - 1.99 (m, 2H), 1.75 - 1.63 (m, 2H).

### 5-{2-[1-(2-Methoxy-ethyl)-1H-pyrazol-4-ylamino]-pyrimidin-4-yl}-2-(tetrahydro-pyran-4-yloxy)-benzonitril ("A35")

In einem 50 mL Kolben, versehen mit Magnetrührer, Kühler und Trockenröhrchen, werden 16 mg 5-[2-(1 H-Pyrazol-4-ylamino)-pyrimidin-4-yl]-2-(tetrahydro-pyran-4-yloxy)-benzonitril in 1 ml getrocknetem Acetonitril gelöst, mit 9 mg Bromethylmethylether und 28 mg Cs₂CO₃ versetzt und die Suspension bei 90°C Badtemperatur gerührt. Das Reaktionsgemisch wird 5 Stunden bei 90°C und über Nacht bei Raumtemperatur gerührt.

Zur Aufarbeitung wird die Mischung einrotiert und mit der präparativen HPLC gereinigt. Man erhält 8 mg vom gewünschten Produkt; HPLC-MS Rt. [min] 1.957
HPLC-MS [M+H] 421;
¹H NMR (500 MHz, DMSO-d₆) δ [ppm] 9.48 (s, 1 H), 8.51 - 8.45 (m, 2H), 8.42 (dd, *J*=9.0, 2.2, 1 H), 7.94 (s, 1H), 7.59 (s, 1H), 7.52 (d, *J*=9.1, 1 H), 7.34 (d, *J*=5.2, 1 H), 4.99 - 4.90 (m, 1H), 4.24 (t, *J*=5.3, 2H), 3.91 - 3.82 (m, 2H), 3.68 (t, *J*=5.3, 2H), 3.56 (ddd, *J*=11.5, 8.4, 3.1, 2H), 3.24 (s, 3H), 2.09 - 1.98 (m, 2H), 1.75 - 1.63 (m, 2H).

### 5-{2-[1-(2-Morpholin-4-yl-ethyl)-1H-pyrazol-4-ylamino]-pyrimidin-4-yl}-2-(tetrahydropyran-4-yloxy)-benzonitril ("A39")

Mit 1-(2-Morpholin-4-yl-ethyl)-1H-pyrazol-4-ylamin erhält man das gewünschte Produkt in 7% Ausbeute; HPLC-MS Rt. [min] 1.537; HPLC-MS [M+H] 476;
¹H NMR (500 MHz, DMSO-d₆) δ [ppm] 9.62 (s, 1H), 8.54 - 8.47 (m, 2H), 8.41 (dd, J=9.0, 2.2, 1 H), 8.06 (s, 1 H), 7.70 (s, 1 H), 7.52 (d, J=9.1, 1 H), 7.36 (d, J=6.2, 1 H), 5.01 - 4.86 (m, 1 H), 4.54 (t, J=6.3, 2H), 3.92 - 3.84 (m, 4H), 3.65 - 3.50 (m, 6H), 3.42 (br, 2H), 3.19 (br, 2H), 2.10 - 1.99 (m, 2H), 1.77 - 1.61 (m, 2H).

### 5-[2-(1-Pyrrolidin-3-yl-1 H-pyrazol-4-ylamino)-pyrimidin-4-yl]-2-(tetrahydro-pyran-4-yloxy)-benzonitril ("A40")

Mit dem oben hergestellten 3-(4-Amino-pyrazol-1-yl)-pyrrolidin-1-carbonsäure-tert.-butylester erhält man 3-(4-{4-[3-Cyan-4-(tetrahydro-pyran-4-yloxy)-phenyl]-pyrimidin-2-ylamino}-pyrazol-1-yl)-pyrrolidin-1-carbonsäure-tert.-butylester in 12% Ausbeute.

41 mg 3-(4-{4-[3-Cyan-4-(tetrahydro-pyran-4-yloxy)-phenyl]-pyrimidin-2-ylamino}-pyrazol-1-yl)-pyrrolidin-1-carbonsäure-tert-butylester werden in 1 mL getrocknetem Dioxan gelöst und mit 1 mL HCl in Dioxan (4 mol/L) versetzt. Die gelbe Lösung wird 60 min bei Raumtemperatur gerührt.

Zur Aufarbeitung wird das Reaktionsgemisch mit 2 molarer NaOH basisch gestellt. Die Lösung wird einrotiert und chromatographiert. Man erhält 22 mg des gewünschten Produkts; HPLC-MS Rt. [min] 1.522; HPLC-MS [M+H] 432;
¹H NMR (500 MHz, DMSO-d₆) δ [ppm] 9.59 (s, 1 H), 9.00 (d, J=21.0, 2H), 8.54 - 8.46 (m, 2H), 8.41 (dd, J=9.0, 2.2, 1 H), 8.08 (s, 1 H), 7.71 (s, 1 H), 7.52 (d, J=9.1, 1 H), 7.36 (d, J=5.2, 1 H), 5.25 - 5.15 (m, 1 H), 4.99 - 4.87 (m, 1 H), 3.92 - 3.80 (m, 3H), 3.67 - 3.52 (m, 6H), 2.46 - 2.33 (m, 1 H), 2.33 - 2.20 (m, 1 H), 2.09 - 1.99 (m, 2H), 1.74 - 1.63 (m, 2H).

### 5-{2-[1-(Tetrahydro-furan-3-ylmethyl)-1H-pyrazol-4-ylamino]-pyrimidin-4-yl}-2-(tetrahydro-pyran-4-yloxy)-benzonitril ("A41")

Mit dem oben hergestellten 1-(Tetrahydro-furan-3-ylmethyl)-1H-pyrazol-4-ylamin erhält man das gewünschte Produkt in 8% Ausbeute; HPLC-MS Rt. [min] 1.986; HPLC-MS [M+H] 447;
¹H NMR (500 MHz, DMSO-d₆) δ [ppm] 9.49 (s, 1H), 8.52 - 8.45 (m, 2H), 8.41 (d, J=8.9, 1 H), 7.96 (s, 1 H), 7.58 (s, 1 H), 7.52 (d, J=9.0, 1 H), 7.33 (d, J=5.2, 1 H), 4.99 - 4.88 (m, 1H), 4.11 - 4.04 (m, 2H), 3.91 - 3.83 (m, 2H), 3.76 (dd, J=13.8, 7.9, 1 H), 3.70 - 3.60 (m, 2H), 3.60 - 3.51 (m, 2H), 3.47 (dd, J=8.3, 5.7, 1 H), 2.76 - 2.65 (m, 1 H), 2.10 - 1.98 (m, 2H), 1.97 - 1.85 (m, 1 H), 1.75 - 1.56 (m, 3H).

### 5-{4-[3-Cyan-4-(tetrahydro-pyran-4-yloxy)-phenyl]-pyrimidin-2-ylamino}-benzofuran-2-carbonsäure amid ("A42")

Mit 5-Amino-benzofuran-2-carbonsäure-amid erhält man das gewünschte Produkt in 5% Ausbeute; HPLC-MS Rt. [min] 2.036; HPLC-MS [M+H] 456;
¹H NMR (500 MHz, DMSO-d₆) δ [ppm] 9.75 (s, 1H), 8.55 (dd, J=8.3, 3.7, 2H), 8.46 (dd, J=9.0, 2.3, 1 H), 8.28 (d, J=2.1, 1 H), 8.03 (s, 1 H), 7.72 (dd, J=9.0, 2.2, 1 H), 7.62 (s, 1 H), 7.56 (dd, J=12.4, 9.1, 2H), 7.51 (d, J=0.6, 1 H), 7.47 (d, J=5.3, 1 H), 5.00 - 4.88 (m, 1 H), 3.93 - 3.83 (m, 2H), 3.60 - 3.51 (m, 2H), 2.10 - 2.00 (m, 2H), 1.76 - 1.61 (m, 2H).

### 5-{2-[1-(2-Pyrazol-1-yl-ethyl)-1H-pyrazol-4-ylamino]-pyrimidin-4-yl}-2-(tetrahydropyran-4-yloxy)-benzonitril ("A43")

Mit dem oben hergestellten 1-(2-Pyrazol-1-yl-ethyl)-1 H-pyrazol-4-ylamin erhält man das gewünschte Produkt in 8% Ausbeute; HPLC-MS Rt. [min] 1.954; HPLC-MS [M+H] 457;
¹H NMR (500 MHz, DMSO-d₆) δ [ppm] 9.47 (s, 1 H), 8.49 - 8.43 (m, 2H), 8.39 (dd, J=9.0, 2.3, 1H), 7.68 (s, 1H), 7.52 (d, J=9.1, 1H), 7.48 (d, J=2.1, 1H), 7.45 - 7.42 (m, 1 H), 7.32 (d, J=5.2, 1 H), 6.20 - 6.13 (m, 1 H), 5.00 - 4.88 (m, 1 H), 4.56 - 4.45 (m, 4H), 3.91 - 3.83 (m, 2H), 3.61 - 3.50 (m, 2H), 2.09 - 1.98 (m, 2H), 1.76 - 1.59 (m, 2H).

### 5-{2-[1-(2,2-Difluor-ethyl)-1H-pyrazol-4-ylamino]-pyrimidin-4-yl}-2-(tetrahydro-pyran-4-yloxy)-benzonitril ("A44")

Mit dem oben hergestellten 1-(2,2-Difluor-ethyl)-1H-pyrazol-4-ylamin erhält man das gewünschte Produkt in 11% Ausbeute; HPLC-MS Rt. [min] 2.069;
HPLC-MS [M+H] 427;
¹H NMR (500 MHz, DMSO-d₆) δ [ppm] 9.56 (s, 1H), 8.53 - 8.46 (m, 2H), 8.42 (dd, *J*=9.0, 2.2, 1 H), 8.03 (s, 1H), 7.66 (s, 1H), 7.51 (d, *J*=9.1, 1 H), 7.35 (d, *J*=5.3, 1 H), 6.34 (tt, *J*=55.1, 3.8, 1H), 5.01 - 4.89 (m, 1 H), 4.60 (td, *J*=15.1, 3.8, 2H), 3.93 - 3.76 (m, 2H), 3.56 (ddd, *J*=11.5, 8.4, 3.1, 2H), 2.10 - 1.91 (m, 2H), 1.77 - 1.62 (m, 2H).

### 5-{2-[1-(2-Piperidin-4-yl-ethyl)-1H-pyrazol-4-ylamino]-pyrimidin-4-yl}-2-(tetrahydropyran-4-yloxy)-benzonitril ("A45")

Mit dem oben hergestellten 4-[2-(4-Amino-pyrazol-1-yl)-ethyl]-piperidin-1-carbonsäure-tert.-butylester erhält man 4-[2-(4-{4-[3-Cyan-4-(tetrahydro-pyran-4-yloxy)-phenyl]-pyrimidin-2-ylamino}-pyrazol-1-yl)-ethyl]-piperidin-1-carbonsäure-tert.-butylester in 27 % Ausbeute.

119 mg 4-[2-(4-{4-[3-Cyan-4-(tetrahydro-pyran-4-yloxy)-phenyl]-pyrimidin-2-ylamino}-pyrazol-1-yl)-ethyl]-piperidin-1-carbonsäure-tert.-butylester werden in 3 mL getrocknetem Dioxan gelöst und mit 3 mL HCl in Dioxan (4mol/L) versetzt. Die gelbe Lösung wird 60 min bei Raumtemperatur gerührt.

Das Reaktionsgemisch wird mit 2 molarer NaOH basisch gestellt und extrahiert. Die vereinten organischen Phasen werden getrocknet, filtriert und eingeengt. Das Rohprodukt wird chromatographiert. Man erhält 91 mg der gewünschten Verbindung; HPLC-MS Rt. [min] 1.556; HPLC-MS [M+H] 474;
¹H NMR (500 MHz, DMSO-d₆) δ [ppm] 9.51 (s, 1H), 8.57 - 8.45 (m, 3H), 8.41 (dd, *J*=9.0, 2.2, 1 H), 8.21 (d, *J*=25.7, 1 H), 7.95 (s, 1H), 7.58 (s, 1H), 7.52 (d, *J*=9.2, 1H), 7.35 (d, *J*=7.5, 1 H), 4.99 - 4.90 (m, 1 H), 4.14 (t, *J*=6.9, 2H), 3.91 - 3.83 (m, 2H), 3.61 - 3.51 (m, 2H), 3.23 (d, *J*=12.7, 2H), 2.81 (q, *J*=12.4, 2H), 2.09 - 2.00 (m, 2H), 1.88 - 1.63 (m, 6H), 1.55 - 1.42 (m, 1H), 1.39 - 1.24 (m, 2H).

### Synthese von 5-{2-[1-(3-Methoxy-propyl)-1H-pyrazol-4-ylamino]-pyrimidin-4-yl}-2-(tetrahydro-pyran-4-yloxy)-benzonitril ("A46")

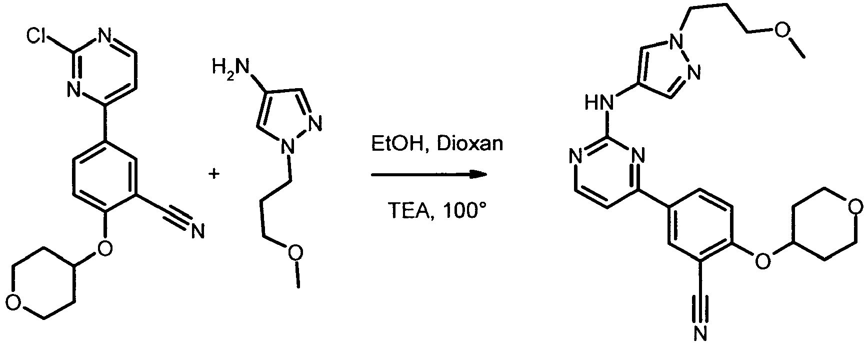

In einem 100 mL Dreihalskolben werden 5-(2-Chlor-pyrimidin-4-yl)-2-(tetrahydropyran-4-yloxy)-benzonitril (200 mg) in Ethanol und Dioxan gelöst und mit dem oben hergestellten 1-(3-Methoxy-propyl)-1H-pyrazol-4-ylamin (129 mg) und 0.8 ml Triethylamin versetzt. Die gelbe Lösung wird bei 100°C zwei Tage gerührt.

Zur Aufarbeitung wird das Gemisch einrotiert und chromatographisch gereinigt. Man erhält 71 mg vom gewünschten Produkt; HPLC-MS Rt. [min] 2.041;
HPLC-MS [M+H] 435;
¹H NMR (500 MHz, DMSO-d₆) δ [ppm] 9.47 (s, 1H), 8.51 - 8.45 (m, 2H), 8.41 (dd, *J*=9.0, 2.3, 1H), 7.91 (s, 1H), 7.58 (s, 1 H), 7.52 (d, *J*=9.1, 1H), 7.33 (d, *J*=5.2, 1 H), 4.99 - 4.88 (m, 1H), 4.12 (t, *J*=6.9, 2H), 3.94 - 3.81 (m, 2H), 3.61 - 3.49 (m, 2H), 3.37 - 3.24 (m, 2H), 3.24 (s, 3H), 2.10 - 1.93 (m, 4H), 1.78 - 1.61 (m, 2H).

### Synthese von 5-{2-[5-(3,6-Dihydro-2H-pyran-4-yl)-pyridin-2-ylamino]-pyridin-4-yl}-2-(tetrahydro-pyran-4-yloxy)-benzonitril ("A47")

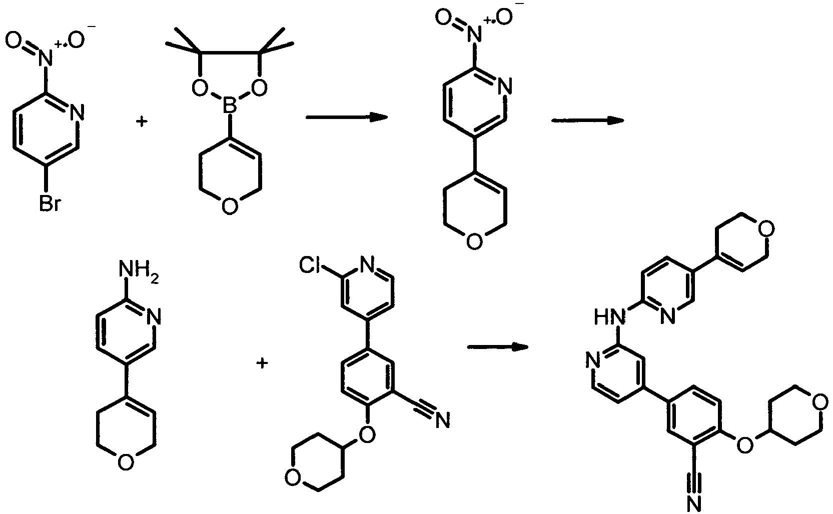

In einem 50 ml Dreihalskolben werden unter N₂ 5-Brom-2-nitro-pyridin (200 mg; 0,985 mmol) und 4-(4,4,5,5-Tetramethyl-[1,3,2]dioxaborolan-2-yl)-3,6-dihydro-2H-pyran (227 mg; 1,084 mmol) in 3 ml Dioxan und 1 ml Wasser gelöst, mit Natriumcarbonat (208 mg; 1,971 mmol) und Bis(triphenylphosphin)-palladium(II)-chlorid (69 mg; 0,099 mmol) versetzt. Das Gemisch wird 1 Stunde auf 80° erhitzt und über Nacht bei Raumtemperatur gerührt.

Zur Aufarbeitung wird das Dioxan einrotiert, der Rückstand mit Wasser verdünnt und mit Dichlormethan extrahiert. Die vereinten organischen Phasen werden mit Wasser gewaschen, getrocknet, filtriert und eingeengt. Der Rückstand wird über eine Kieselgelsäule (Petrolether/Ethylacetat 1/1) gereinigt. Man erhält 174 mg 5-(3,6-Dihydro-2H-pyran-4-yl)-2-nitro-pyridin; HPLC-MS Rt. [min] 1.665;
HPLC-MS [M+H] 207.

174 mg 5-(3,6-Dihydro-2H-pyran-4-yl)-2-nitro-pyridin werden mit 100 mg Pd-C-5% und Wasserstoff in 10 ml Tetrahydrofuran hydriert. Das Gemisch wird abfiltriert und einrotiert. Man erhält 138 mg 5-(3,6-Dihydro-2H-pyran-4-yl)-pyridin-2-ylamin Rohprodukt, das oben Aufreinigung weiter umgesetzt wird.

Mit 5-(2-Chlor-pyridin-4-yl)-2-(tetrahydro-pyran-4-yloxy)-benzonitril und dem hergestellten 5-(3,6-Dihydro-2H-pyran-4-yl)-pyridin-2-ylamin erhält man unter den angegebenen Buchwald-Hartwig-Bedingungen 5-{2-[5-(3,6-Dihydro-2H-pyran-4-yl)-pyridin-2-ylamino]-pyridin-4-yl}-2-(tetrahydro-pyran-4-yloxy)-benzonitril in 23% Ausbeute; HPLC-MS Rt. [min] 1.717; HPLC-MS [M+H] 455;
¹H NMR (500 MHz, DMSO-d₆) δ [ppm] 11.19 (s, 1H), 8.38 (dd, *J*=9.1, 6.2, 2H), 8.24 - 8.21 (m, 1H), 8.13 - 8.04 (m, 2H), 7.68 (s, 1H), 7.57 (d, *J*=9.1, 1H), 7.54 - 7.45 (m, 2H), 6.37 (s, 1 H), 4.99 - 4.89 (m, 1H), 4.29 - 4.20 (m, 2H), 3.91 - 3.81 (m, 5H), 3.61 - 3.52 (m, 2H), 2.47 (m, 1 H), 2.09 - 2.00 (m, 2H), 1.75 - 1.64 (m, 2H).

### 5-[2-(1',2',3',6'-Tetrahydro-[3,4']bipyridinyl-6-ylamino)-pyridin-4-yl]-2-(tetrahydropyran-4-yloxy)-benzonitril ("A48")

Ausgehend von 4-(4,4,5,5-Tetramethyl-[1,3,2]dioxaborolan-2-yl)-3,6-dihydro-2H-pyridin-1-carbonsäure-tert.-butylester erhält man in gleicher Reaktionssequenz 6-{4-[3-Cyan-4-(tetrahydro-pyran-4-yloxy)-phenyl]-pyridin-2-ylamino}-3',6'-dihydro-2'H-[3,4']bipyridinyl-1'-carbonsäure-tert--butylester.

6-{4-[3-Cyan-4-(tetrahydro-pyran-4-yloxy)-phenyl]-pyridin-2-ylamino}-3',6'-dihydro-2'H-[3,4']bipyridinyl-1'-carbonsäure-tert.-butylester (247 mg; 0,134 mmol) werden in 2 mL getrocknetem Dioxan gelöst und mit 2 mL HCl in Dioxan (4mol/L) versetzt. Das Reaktionsgemisch wird 1 h bei Raumtemperatur gerührt.

Zur Aufarbeitung wird das Reaktionsgemisch mit 2 molarer NaOH basisch gestellt. Die Lösung wird dann einrotiert und mit Dichloromethan versetzt. Die organischen Phasen werden getrocknet, filtriert und eingeengt. Das Rohprodukt wird chromatographisch gereinigt. Man erhält das gewünschte Produkt in 20% Ausbeute; HPLC-MS Rt. [min] 1.352; HPLC-MS [M+H] 454;
¹H NMR (500 MHz, DMSO-d₆) δ [ppm] 10.67 (br, 1H), 8.79 (br, 2H), 8.41 (d, *J*=2.4, 1H), 8.34 (d, *J*=5.7, 1 H), 8.17 (m, 1H), 8.06 - 7.96 (m, 2H), 7.84 (s, 1H), 7.64 (d, *J*=8.7, 1H), 7.58 - 7.52 (m, 1H), 7.41 (d, *J*=4.5, 1H), 6.23 (s, 1H), 5.01 - 4.86 (m, 1H), 3.93 - 3.83 (m, 3H), 3.79 (s, 2H), 3.61 - 3.50 (m, 2H), 3.39 - 3.31 (m, 2H), 2.70 (s, 1 H), 2.09 - 1.97 (m, 2H), 1.74 - 1.62 (m, 2H).

Analog werden die nachstehenden Verbindungen hergestellt

| Verbindung Nr. | Struktur und/oder Name | |
|---|---|---|
| "A53" | 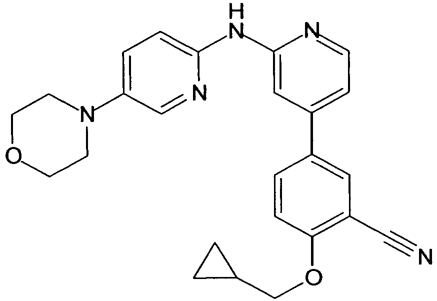 | |
| "A58" | 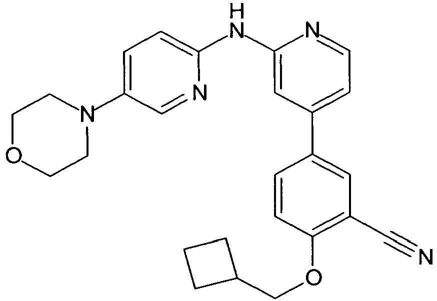 | |
| "A59" | 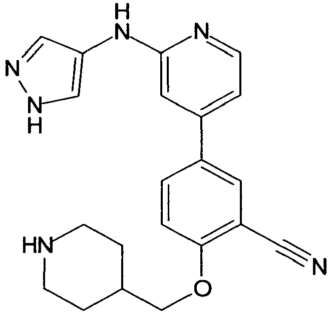 | |
| "A60" | 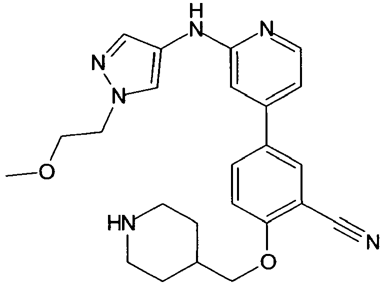 | |
| "A61" | 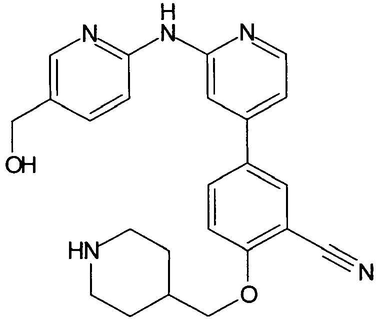 | |
| "A62" | 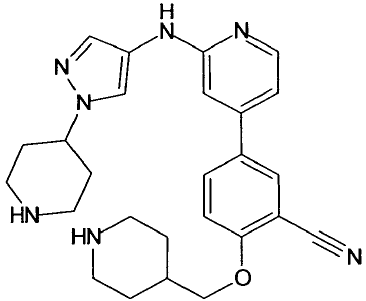 | |
| "A63" | 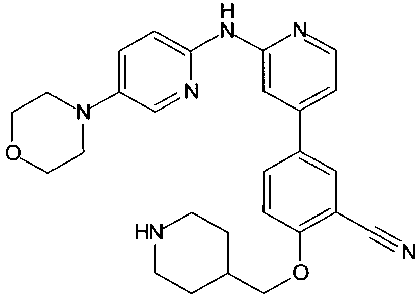 | |

IC₅₀-Werte von erfindungsgemäßen TBK1- und IKKε-hemmenden Verbindungen

| Verbindung Nr. | TBK1-Enzymassay IC₅₀ [nM] | IKKε-Enzymassay IC₅₀ [nM] | TBK1 + IKKε-Zellassay IC₅₀ [nM] |
|---|---|---|---|
| "A1" | | | |
| "A2" | 83 | 20 | 960 |
| "A3" | 260 | 370 | 3300 |
| "A4" | 34 | 38 | 6600 |
| "A5" | 97 | 110 | 2900 |
| "A6" | | | |
| "A7" | 8 | 15 | 350 |
| "A8" | | | |
| "A9" | 120 | 100 | 3000 |
| "A10" | 21 | 43 | 1200 |
| "A11" | 250 | 240 | 5200 |
| "A12" | 30 | 25 | 1300 |
| "A13" | 310 | 530 | |
| "A14" | 670 | 1200 | |
| "A15" | 14 | 21 | 1100 |
| "A16" | 71 | 7 | 5000 |
| "A17" | | | |
| "A18" | 8 | 13 | |
| "A19" | 1900 | 390 | |
| "A20" | 55 | 51 | 4800 |
| "A21" | 70 | 37 | 5300 |
| "A22" | 100 | 150 | 9600 |
| "A23" | 120 | 160 | 7800 |
| "A24" | 110 | 89 | 4800 |
| "A25" | 290 | 67 | |
| "A26" | 18 | 27 | 3300 |
| "A27" | 85 | 77 | 5000 |
| "A28" | 280 | 74 | 23000 |
| "A29" | 120 | 88 | 6700 |
| "A30" | 42 | 45 | |
| "A31" | 860 | 850 | 19000 |
| "A32" | | | |
| "A33" | 130 | 58 | 5000 |
| "A34" | 6 | 6 | 370 |
| "A35" | 6 | 18 | 670 |
| "A36" | | 140 | |
| "A37" | 32 | 21 | |
| "A38" | 460 | 320 | |
| "A39" | 16 | 25 | |
| "A40" | 7 | 5 | |
| "A41" | 12 | 25 | |
| "A42" | 10 | 15 | |
| "A43" | 70 | 65 | |
| "A44" | 5 | 10 | |
| "A45" | 2 | 2 | |

Die nachfolgenden Beispiele betreffen Arzneimittel:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Verbindung Nr. | Name und/oder Struktur |
|---|---|
| "A1" | 2-(Tetrahydro-pyran-4-yloxy)-5-{2-[1-(3-trifluormethyl-phenyl)-1H-pyrazol-4-ylamino]-pyridin-4-yl}-benzonitril |
| "A2" | 5-{2-[1-(1-Methyl-piperidin-4-yl)-1H-pyrazol-4-ylamino]-pyridin-4-yl}-2-(tetrahydro-pyran-4-yloxy)-benzonitril |
| "A3" | 5-[2-([3,3']Bipyridinyl-6-ylamino)-pyridin-4-yl]-2-(tetrahydropyran-4-yloxy)-benzonitril |
| "A4" | 5-[2-(5-Methyl-isoxazol-3-ylamino)-pyridin-4-yl]-2-(tetrahydropyran-4-yloxy)-benzonitril |
| "A5" | 5-[2-(1-Methyl-1 H-pyrazol-3-ylamino)-pyridin-4-yl]-2-(tetrahydropyran-4-yloxy)-benzonitril |
| "A6" | 5-[2-(2-Furan-2-ylmethyl-2H-pyrazol-3-ylamino)-pyridin-4-yl]-2-(tetrahydro-pyran-4-yloxy)-benzonitril |
| "A7" | 5-[2-(5-Morpholin-4-yl-pyridin-2-ylamino)-pyridin-4-yl]-2-(tetrahydro-pyran-4-yloxy)-benzonitril |
| "A8" | 5-[2-(1-Phenyl-1 H-pyrazol-4-ylamino)-pyridin-4-yl]-2-(tetrahydropyran-4-yloxy)-benzonitril |
| "A9" | 5-{2-[5-(1H-Pyrazol-4-yl)-pyridin-2-ylamino]-pyridin-4-yl}-2-(tetrahydro-pyran-4-yloxy)-benzonitril |
| "A10" | 5-[2-(5-tert.-Butyl-1 H-pyrazol-3-ylamino)-pyridin-4-yl]-2-(tetrahydro-pyran-4-yloxy)-benzonitril |
| "A11" | 6-{4-[3-Cyan-4-(tetrahydro-pyran-4-yloxy)-phenyl]-pyridin-2-ylamino}-nicotinonitril |
| "A12" | 5-[2-(5-Cyclopropyl-2H-pyrazol-3-ylamino)-pyridin-4-yl]-2-(tetrahydro-pyran-4-yloxy)-benzonitril |
| "A13" | 2-(Tetrahydro-pyran-4-yloxy)-5-[2-(5-trifluormethyl-pyridin-2-ylamino)-pyridin-4-yl]-benzonitril |
| "A14" | 5-[2-(Pyrimidin-2-ylamino)-pyridin-4-yl]-2-(tetrahydro-pyran-4-yloxy)-benzonitril |
| "A15" | 5-[2-(5-Hydroxymethyl-pyridin-2-ylamino)-pyridin-4-yl]-2-(tetrahydro-pyran-4-yloxy)-benzonitril |
| "A16" | 5-[2-(1-Piperidin-4-yl-1 H-pyrazol-4-ylamino)-pyridin-4-yl]-2-(tetrahydro-pyran-4-yloxy)-benzonitril |
| "A17" | 2-{4-[3-Cyan-4-(tetrahydro-pyran-4-yloxy)-phenyl]-pyridin-2-ylamino}-isonicotinonitril |
| "A18" | 5-[2-(4-Hydroxymethyl-pyridin-2-ylamino)-pyridin-4-yl]-2-(tetrahydro-pyran-4-yloxy)-benzonitril |
| "A19" | 5-{4-[3-Cyan-4-(tetrahydro-pyran-4-yloxy)-phenyl]-pyridin-2-ylamino}-benzofuran-2-carbonsäure-amid |
| "A20" | 5-{2-[1-(2,2-Difluor-ethyl)-1H-pyrazol-4-ylamino]-pyridin-4-yl}-2-(tetrahydro-pyran-4-yloxy)-benzonitril |
| "A21" | 5-{2-[1-(2-Piperidin-4-yl-ethyl)-1H-pyrazol-4-ylamino]-pyridin-4-yl}-2-(tetrahydro-pyran-4-yloxy)-benzonitril |
| "A22" | 5-{2-[1-(2-Morpholin-4-yl-ethyl)-1H-pyrazol-4-ylamino]-pyridin-4-yl}-2-(tetrahydro-pyran-4-yloxy)-benzonitril |
| "A23" | 5-{2-[1-(3-Methoxy-propyl)-1H-pyrazol-4-ylamino]-pyridin-4-yl}-2-(tetrahydro-pyran-4-yloxy)-benzonitril |
| "A24" | 5-{2-[1-(2-Cyan-cyclopropylmethyl)-1H-pyrazol-4-ylamino]-pyridin-4-yl}-2-(tetrahydro-pyran-4-yloxy)-benzonitril |
| "A25" | 5-[2-(1-Azetidin-3-yl-1 H-pyrazol-4-ylamino)-pyridin-4-yl]-2-(tetrahydro-pyran-4-yloxy)-benzonitril |
| "A26" | 5-{2-[1-((1S,2S)-2-Hydroxymethyl-cyclopropylmethyl)-1H-pyrazol-4-ylamino]-pyridin-4-yl}-2-(tetrahydro-pyran-4-yloxy)-benzonitril |
| "A27" | 5-{2-[1-(Tetrahydro-furan-3-ylmethyl)-1H-pyrazol-4-ylamino]-pyridin-4-yl}-2-(tetrahydro-pyran-4-yloxy)-benzonitril |
| "A28" | 5-[2-(1-Pyrrolidin-3-yl-1H-pyrazol-4-ylamino)-pyridin-4-yl]-2-(tetrahydro-pyran-4-yloxy)-benzonitril |
| "A29" | 5-[2-(1-{2-[1-(2-Hydroxy-acetyl)-piperidin-4-yl]-ethyl}-1H-pyrazol-4-ylamino)-pyridin-4-yl]-2-(tetrahydro-pyran-4-yloxy)-benzonitril |
| "A30" | 5-[2-(1-{2-[1-(2-Amino-acetyl)-piperidin-4-yl]-ethyl}-1H-pyrazol-4-ylamino)-pyridin-4-yl]-2-(tetrahydro-pyran-4-yloxy)-benzonitril |
| "A31" | 5-[2-(3-tert.-Butyl-isoxazol-5-ylamino)-pyridin-4-yl]-2-(tetrahydropyran-4-yloxy)-benzonitril |
| "A32" | 2-(Tetrahydro-pyran-4-yloxy)-5-{2-[1-(3-trifluormethyl-phenyl)-1H-pyrazol-4-ylamino]-pyrimidin-4-yl}-benzonitril |
| "A33" | 5-[2-(1-Methyl-1H-pyrazol-3-ylamino)-pyrimidin-4-yl]-2-(tetrahydro-pyran-4-yloxy)-benzonitril |
| "A34" | 5-[2-(1 H-Pyrazol-4-ylamino)-pyrimidin-4-yl]-2-(tetrahydro-pyran-4-yloxy)-benzonitril |
| "A35" | 5-{2-[1-(2-Methoxy-ethyl)-1H-pyrazol-4-ylamino]-pyrimidin-4-yl}-2-(tetrahydro-pyran-4-yloxy)-benzonitril |
| "A36" | 2-(Tetrahydro-pyran-4-yloxy)-5-[2-(5,6,7,8-tetrahydro-pyrido[4,3-d]pyrimidin-2-ylamino)-pyridin-4-yl]-benzonitril |
| "A37" | 6-{4-[3-Cyan-4-(tetrahydro-pyran-4-yloxy)-phenyl]-pyridin-2-ylamino}-nicotinamid |
| "A38" | 5-{2-[1-(2-Pyrazol-1-yl-ethyl)-1H-pyrazol-4-ylamino]-pyridin-4-yl}-2-(tetrahydro-pyran-4-yloxy)-benzonitril |
| "A39" | 5-{2-[1-(2-Morpholin-4-yl-ethyl)-1H-pyrazol-4-ylamino]-pyrimidin-4-yl}-2-(tetrahydro-pyran-4-yloxy)-benzonitril |
| "A40" | 5-[2-(1-Pyrrolidin-3-yl-1 H-pyrazol-4-ylamino)-pyrimidin-4-yl]-2-(tetrahydro-pyran-4-yloxy)-benzonitril |
| "A41" | 5-{2-[1-(Tetrahydro-furan-3-ylmethyl)-1H-pyrazol-4-ylamino]-pyrimidin-4-yl}-2-(tetrahydro-pyran-4-yloxy)-benzonitril |
| "A42" | 5-{4-[3-Cyan-4-(tetrahydro-pyran-4-yloxy)-phenyl]-pyrimidin-2-ylamino}-benzofuran-2-carbonsäure amid |
| "A43" | 5-{2-[1-(2-Pyrazol-1-yl-ethyl)-1H-pyrazol-4-ylamino]-pyrimidin-4-yl}-2-(tetrahydro-pyran-4-yloxy)-benzonitril |
| "A44" | 5-{2-[1-(2,2-Difluor-ethyl)-1H-pyrazol-4-ylamino]-pyrimidin-4-yl}-2-(tetrahydro-pyran-4-yloxy)-benzonitril |
| "A45" | 5-{2-[1-(2-Piperidin-4-yl-ethyl)-1H-pyrazol-4-ylamino]-pyrimidin-4-yl}-2-(tetrahydro-pyran-4-yloxy)-benzonitril |
| "A46" | 5-{2-[1-(3-Methoxy-propyl)-1H-pyrazol-4-ylamino]-pyrimidin-4-yl}-2-(tetrahydro-pyran-4-yloxy)-benzonitril |
| "A47" | 5-{2-[5-(3,6-Dihydro-2H-pyran-4-yl)-pyridin-2-ylamino]-pyridin-4-yl}-2-(tetrahydro-pyran-4-yloxy)-benzonitril |
| "A48" | 5-[2-(1',2',3',6'-Tetrahydro-[3,4']bipyridinyl-6-ylamino)-pyridin-4-yl]-2-(tetrahydro-pyran-4-yloxy)-benzonitril |
| "A53" | 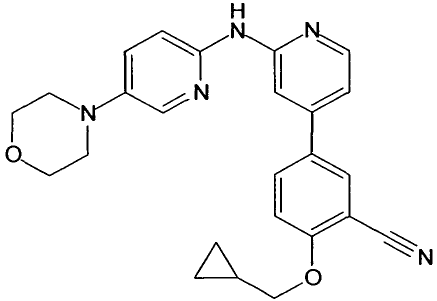 |
| "A58" | 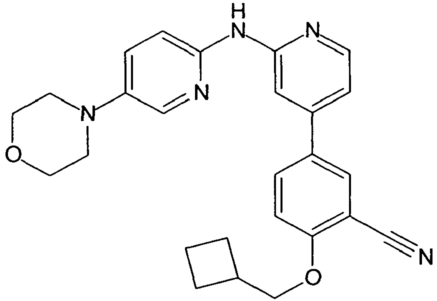 |
| "A59" | 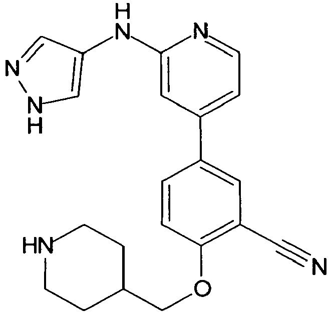 |
| "A60" | 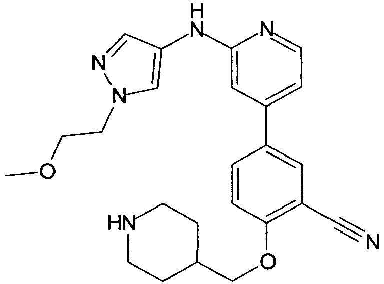 |
| "A61" | 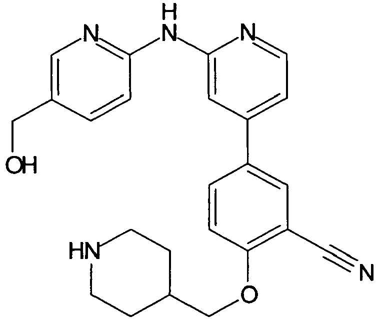 |
| "A62" | 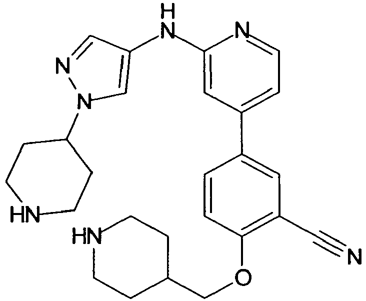 |
| "A63" | 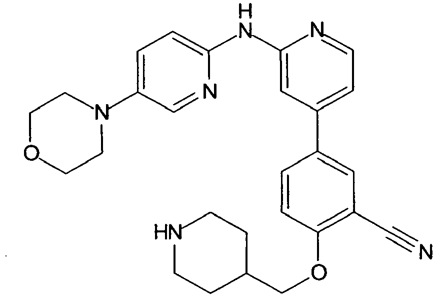 |
sowie ihre pharmazeutisch verwendbaren Salze, Tautomere und Stereoisomere, einschließlich deren Mischungen in allen Verhältnissen.

2. Arzneimittel enthaltend mindestens eine Verbindung gemäß Anspruch 1 und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

3. Verbindungen gemäß Anspruch 1 sowie ihre pharmazeutisch verwendbaren Salze, Tautomere und Stereoisomere, einschließlich deren Mischungen in allen Verhältnissen, zur Verwendung für die Behandlung von Krebs, septischem Schock, primärem Offenwinkelglaukom (POAG), Hyperplasie, Atherosklerose, Retinopathie, Osteoarthritis, Endometriose, chronischer Entzündung, neurodegenerativen Erkrankungen, rheumatoide Arthritis (RA), systemischer Lupus erythematosus (SLE), Sjörgrens Syndrom, Aicardi-Goutieres Syndrom Lupus Chilblain, retinale Vasculopathie, cerebrale Leukodystrophie (RVCL), systemische Sklerosis, Myositis, Psoriasis, chronisch obstruktive pulmonare Krankheit (CPD), endzündliche Darmkrankheit (IBD), Fettsucht, Insulinresistenz, Typ 2 Diabetes (NIDDM) und/oder metabolisches Syndrom.

4. Verbindungen gemäß Anspruch 1 und/oder ihre physiologisch unbedenklichen Salze, Tautomere und Stereoisomere zur Verwendung für die Behandlung von Tumoren, wobei eine therapeutisch wirksame Menge einer Verbindung gemäß Anspruch 1 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weitere Angiogenese-Hemmer verabreicht wird.

5. Verbindungen gemäß Anspruch 1 und/oder ihre physiologisch unbedenklichen Salze, Tautomere und Stereoisomere zur Verwendung für die Behandlung von Tumoren, wobei eine therapeutisch wirksame Menge einer Verbindung gemäß Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weitere Angiogenese-Hemmer verabreicht wird.

## Claims

1. Compounds selected from the group
| Compound No. | Name and/or structure |
|---|---|
| "A1" | 2-(Tetrahydropyran-4-yloxy)-5-{2-[1-(3-trifluoromethylphenyl)-1H-pyrazol-4-ylamino]pyridin-4-yl}benzonitrile |
| "A2" | 5-{2-[1-(1-Methylpiperidin-4-yl)-1H-pyrazol-4-ylamino]pyridin-4-yl}-2-(tetrahydropyran-4-yloxy)benzonitrile |
| "A3" | 5-[2-([3,3']Bipyridinyl-6-ylamino)pyridin-4-yl]-2-(tetrahydropyran-4-yloxy)benzonitrile |
| "A4" | 5-[2-(5-Methylisoxazol-3-ylamino)pyridin-4-yl]-2-(tetrahydropyran-4-yloxy)benzonitrile |
| "A5" | 5-[2-(1-Methyl-1H-pyrazol-3-ylamino)pyridin-4-yl]-2-(tetrahydropyran-4-yloxy)benzonitrile |
| "A6" | 5-[2-(2-Furan-2-ylmethyl-2H-pyrazol-3-ylamino)pyridin-4-yl]-2-(tetrahydropyran-4-yloxy)benzonitrile |
| "A7" | 5-[2-(5-Morpholin-4-ylpyridin-2-ylamino)pyridin-4-yl]-2-(tetrahydropyran-4-yloxy)benzonitrile |
| "A8" | 5-[2-(1-Phenyl-1H-pyrazol-4-ylamino)pyridin-4-yl]-2-(tetrahydropyran-4-yloxy)benzonitrile |
| "A9" | 5-{2-[5-(1H-Pyrazol-4-yl)pyridin-2-ylamino]pyridin-4-yl}-2-(tetrahydropyran-4-yloxy)benzonitrile |
| "A10" | 5-[2-(5-tert-Butyl-1 H-pyrazol-3-ylamino)pyridin-4-yl]-2-(tetrahydropyran-4-yloxy)benzonitrile |
| "A11" | 6-{4-[3-Cyano-4-(tetrahydropyran-4-yloxy)phenyl]pyridin-2-yl-amino}nicotinonitrile |
| "A12" | 5-[2-(5-Cyclopropyl-2H-pyrazol-3-ylamino)pyridin-4-yl]-2-(tetrahydropyran-4-yloxy)benzonitrile |
| "A13" | 2-(Tetrahydropyran-4-yloxy)-5-[2-(5-trifluoromethylpyridin-2-yl-amino)pyridin-4-yl]benzonitrile |
| "A14" | 5-[2-(Pyrimidin-2-ylamino)pyridin-4-yl]-2-(tetrahydropyran-4-yl-oxy)benzonitrile |
| "A15" | 5-[2-(5-Hydroxymethylpyridin-2-ylamino)pyridin-4-yl]-2-(tetrahydropyran-4-yloxy)benzonitrile |
| "A16" | 5-[2-(1-Piperidin-4-yl-1H-pyrazol-4-ylamino)pyridin-4-yl]-2-(tetrahydropyran-4-yloxy)benzonitrile |
| "A17" | 2-{4-[3-Cyano-4-(tetrahydropyran-4-yloxy)phenyl]pyridin-2-yl-amino}isonicotinonitrile |
| "A18" | 5-[2-(4-Hydroxymethylpyridin-2-ylamino)pyridin-4-yl]-2-(tetrahydropyran-4-yloxy)benzonitrile |
| "A19" | 5-{4-[3-Cyano-4-(tetrahydropyran-4-yloxy)phenyl]pyridin-2-yl-amino}benzofuran-2-carboxamide |
| "A20" | 5-{2-[1-(2,2-Difluoroethyl)-1H-pyrazol-4-ylamino]pyridin-4-yl}-2-(tetrahydropyran-4-yloxy)benzonitrile |
| "A21" | 5-{2-[1-(2-Piperidin-4-ylethyl)-1H-pyrazol-4-ylamino]pyridin-4-yl}-2-(tetrahydropyran-4-yloxy)benzonitrile |
| "A22" | 5-{2-[1-(2-Morpholin-4-ylethyl)-1H-pyrazol-4-ylamino]pyridin-4-yl}-2-(tetrahydropyran-4-yloxy)benzonitrile |
| "A23" | 5-{2-[1-(3-Methoxypropyl)-1H-pyrazol-4-ylamino]pyridin-4-yl}-2-(tetrahydropyran-4-yloxy)benzonitrile |
| "A24" | 5-{2-[1-(2-Cyanocyclopropylmethyl)-1H-pyrazol-4-ylamino]-pyridin-4-yl}-2-(tetrahydropyran-4-yloxy)benzonitrile |
| "A25" | 5-[2-(1-Azetidin-3-yl-1H-pyrazol-4-ylamino)pyridin-4-yl]-2-(tetrahydropyran-4-yloxy)benzonitrile |
| "A26" | 5-{2-[1-((1S,2S)-2-Hydroxymethylcyclopropylmethyl)-1H-pyrazol-4-ylamino]pyridin-4-yl}-2-(tetrahydropyran-4-yloxy)-benzonitrile |
| "A27" | 5-{2-[1-(Tetrahydrofuran-3-ylmethyl)-1H-pyrazol-4-ylamino]-pyridin-4-yl}-2-(tetrahydropyran-4-yloxy)benzonitrile |
| "A28" | 5-[2-(1-Pyrrolidin-3-yl-1H-pyrazol-4-ylamino)pyridin-4-yl]-2-(tetrahydropyran-4-yloxy)benzonitrile |
| "A29" | 5-[2-(1-{2-[1-(2-Hydroxyacetyl)piperidin-4-yl]ethyl}-1H-pyrazol-4-ylamino)pyridin-4-yl]-2-(tetrahydropyran-4-yloxy)benzonitrile |
| "A30" | 5-[2-(1-{2-[1-(2-Aminoacetyl)piperidin-4-yl]ethyl}-1H-pyrazol-4-ylamino)pyridin-4-yl]-2-(tetrahydropyran-4-yloxy)benzonitrile |
| "A31" | 5-[2-(3-tert-Butylisoxazol-5-ylamino)pyridin-4-yl]-2-(tetrahydropyran-4-yloxy)benzonitrile |
| "A32" | 2-(Tetrahydropyran-4-yloxy)-5-{2-[1-(3-trifluoromethylphenyl)-1H-pyrazol-4-ylamino]pyrimidin-4-yl}benzonitrile |
| "A33" | 5-[2-(1-Methyl-1H-pyrazol-3-ylamino)pyrimidin-4-yl]-2-(tetrahydropyran-4-yloxy)benzonitrile |
| "A34" | 5-[2-(1H-Pyrazol-4-ylamino)pyrimidin-4-yl]-2-(tetrahydropyran-4-yloxy)benzonitrile |
| "A35" | 5-{2-[1-(2-Methoxyethyl)-1H-pyrazol-4-ylamino]pyrimidin-4-yl}-2-(tetrahydropyran-4-yloxy)benzonitrile |
| "A36" | 2-(Tetrahydropyran-4-yloxy)-5-[2-(5,6,7,8-tetrahydropyrido-[4,3-d]pyrimidin-2-ylamino)pyridin-4-yl]benzonitrile |
| "A37" | 6-{4-[3-Cyano-4-(tetrahydropyran-4-yloxy)phenyl]pyridin-2-yl-amino}nicotinamide |
| "A38" | 5-{2-[1-(2-Pyrazol-1-ylethyl)-1H-pyrazol-4-ylamino]pyridin-4-yl}-2-(tetrahydropyran-4-yloxy)benzonitrile |
| "A39" | 5-{2-[1-(2-Morpholin-4-ylethyl)-1H-pyrazol-4-ylamino]pyrimidin-4-yl}-2-(tetrahydropyran-4-yloxy)benzonitrile |
| "A40" | 5-[2-(1-Pyrrolidin-3-yl-1H-pyrazol-4-ylamino)pyrimidin-4-yl]-2-(tetrahydropyran-4-yloxy)benzonitrile |
| "A41" | 5-{2-[1-(Tetrahydrofuran-3-ylmethyl)-1H-pyrazol-4-ylamino]-pyrimidin-4-yl}-2-(tetrahydropyran-4-yloxy)benzonitrile |
| "A42" | 5-{4-[3-Cyano-4-(tetrahydropyran-4-yloxy)phenyl]pyrimidin-2-yl-amino}benzofuran-2-carboxamide |
| "A43" | 5-{2-[1-(2-Pyrazol-1-ylethyl)-1H-pyrazol-4-ylamino]pyrimidin-4-yl}-2-(tetrahydropyran-4-yloxy)benzonitrile |
| "A44" | 5-{2-[1-(2,2-Difluoroethyl)-1H-pyrazol-4-ylamino]pyrimidin-4-yl}-2-(tetrahydropyran-4-yloxy)benzonitrile |
| "A45" | 5-{2-[1-(2-Piperidin-4-ylethyl)-1H-pyrazol-4-ylamino]pyrimidin-4-yl}-2-(tetrahydropyran-4-yloxy)benzonitrile |
| "A46" | 5-{2-[1-(3-Methoxypropyl)-1H-pyrazol-4-ylamino]pyrimidin-4-yl}-2-(tetrahydropyran-4-yloxy)benzonitrile |
| "A47" | 5-{2-[5-(3,6-Dihydro-2H-pyran-4-yl)pyridin-2-ylamino]pyridin-4-yl}-2-(tetrahydropyran-4-yloxy)benzonitrile |
| "A48" | 5-[2-(1',2',3',6'-Tetrahydro-[3,4']bipyridinyl-6-ylamino)pyridin-4-yl]-2-(tetrahydropyran-4-yloxy)benzonitrile |
| "A53" | 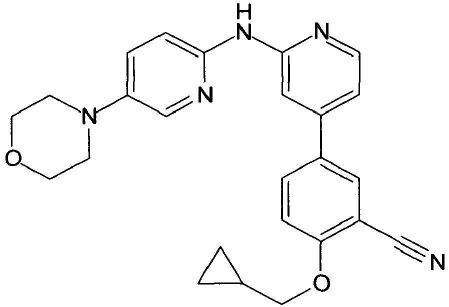 |
| "A58" | 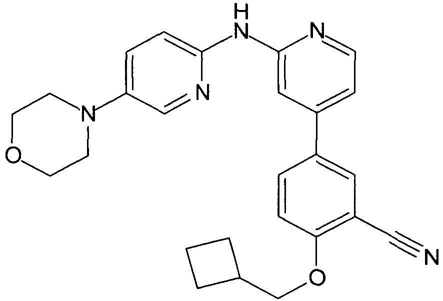 |
| "A59" | 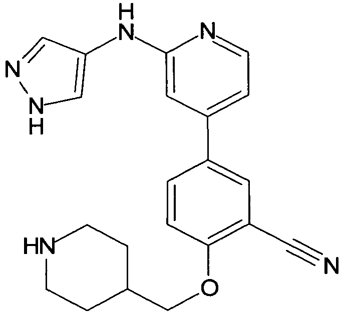 |
| "A60" | 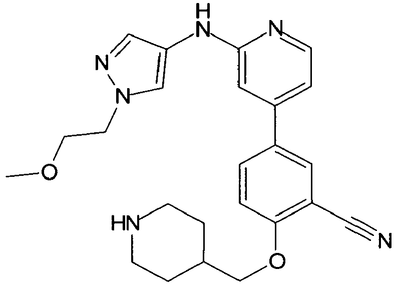 |
| "A61" | 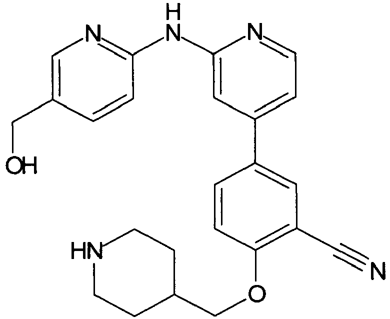 |
| "A62" | 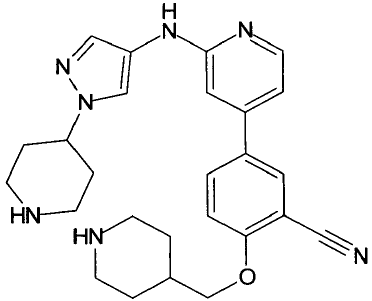 |
| "A63" | 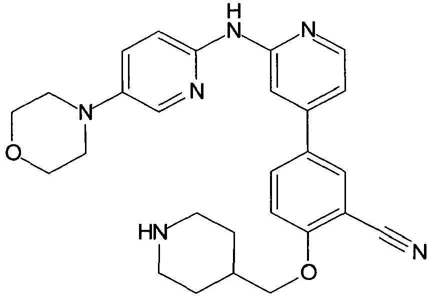 |
and pharmaceutically usable salts, tautomers and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Compounds according to Claim 1 and pharmaceutically usable salts, tautomers and stereoisomers thereof, including mixtures thereof in all ratios, for use for the treatment of cancer, septic shock, primary open angle glaucoma (POAG), hyperplasia, atherosclerosis, retinopathy, osteoarthritis, endometriosis, chronic inflammation, neurodegenerative diseases, rheumatoid arthritis (RA), systemic lupus erythematosus (SLE), Sjörgren's syndrome, Aicardi-Goutières syndrome, chilblain lupus, retinal vasculopathy, cerebral leukodystrophy (RVCL), systemic sclerosis, myositis, psoriasis, chronic obstructive pulmonary disease (CPD), inflammatory bowel disease (IBD), obesity, insulin resistance, type 2 diabetes (NIDDM) and/or metabolic syndrome.

4. Compounds according to Claim 1 and/or physiologically acceptable salts, tautomers and stereoisomers thereof for use for the treatment of tumours, where a therapeutically effective amount of a compound according to Claim 1 is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

5. Compounds according to Claim 1 and/or physiologically acceptable salts, tautomers and stereoisomers thereof for use for the treatment of tumours, where a therapeutically effective amount of a compound according to Claim 1 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés choisis dans le groupe constitué par
| Composé n° | Nom et/ou structure |
|---|---|
| « A1 » | 2-(Tétrahydropyran-4-yloxy)-5-{2-[1-(3-trifluorométhylphényl)-1H-pyrazol-4-ylamino]pyridin-4-yl}benzonitrile |
| « A2 » | 5-{2-[1-(1-Méthylpipéridin-4-yl)-1H-pyrazol-4-ylamino]pyridin-4-yl}-2-(tétrahydropyran-4-yloxy)benzonitrile |
| « A3 » | 5-[2-([3,3']Bipyridinyl-6-ylamino)pyridin-4-yl]-2-(tétrahydropyran-4-yloxy)benzonitrile |
| « A4 » | 5-[2-(5-Méthylisoxazol-3-ylamino)pyridin-4-yl]-2-(tétrahydropyran-4-yloxy)benzonitrile |
| « A5 » | 5-[2-(1-Méthyl-1H-pyrazol-3-ylamino)pyridin-4-yl]-2-(tétrahydropyran-4-yloxy)benzonitrile |
| « A6 » | 5-[2-(2-Furan-2-ylméthyl-2H-pyrazol-3-ylamino)pyridin-4-yl]-2-(tétrahydropyran-4-yloxy)benzonitrile |
| « A7 » | 5-[2-(5-Morpholin-4-ylpyridin-2-ylamino)pyridin-4-yl]-2-(tétrahydropyran-4-yloxy)benzonitrile |
| « A8 » | 5-[2-(1-Phényl-1H-pyrazol-4-ylamino)pyridin-4-yl]-2-(tétrahydropyran-4-yloxy)benzonitrile |
| « A9 » | 5-{2-[5-(1H-Pyrazol-4-yl)pyridin-2-ylamino]pyridin-4-yl}-2-(tétrahydropyran-4-yloxy)benzonitrile |
| « A10 » | 5-[2-(5-tertio-Butyl-1H-pyrazol-3-ylamino)pyridin-4-yl]-2-(tétrahydropyran-4-yloxy)benzonitrile |
| « A11 » | 6-{4-[3-Cyano-4-(tétrahydropyran-4-yloxy)phényl]pyridin-2-yl-amino}nicotinonitrile |
| « A12 » | 5-[2-(5-Cyclopropyl-2H-pyrazol-3-ylamino)pyridin-4-yl]-2-(tétrahydropyran-4-yloxy)benzonitrile |
| « A13 » | 2-(Tétrahydropyran-4-yloxy)-5-[2-(5-trifluorométhylpyridin-2-yl-amino)pyridin-4-yl]benzonitrile |
| « A14 » | 5-[2-(Pyrimidin-2-ylamino)pyridin-4-yl]-2-(tétrahydropyran-4-yl-oxy)benzonitrile |
| « A15 » | 5-[2-(5-Hydroxyméthylpyridin-2-ylamino)pyridin-4-yl]-2-(tétrahydropyran-4-yloxy)benzonitrile |
| « A16 » | 5-[2-(1-Pipéridin-4-yl-1H-pyrazol-4-ylamino)pyridin-4-yl]-2-(tétrahydropyran-4-yloxy)benzonitrile |
| « A17 » | 2-{4-[3-Cyano-4-(tétrahydropyran-4-yloxy)phényl]pyridin-2-yl-amino}isonicotinonitrile |
| « A18 » | 5-[2-(4-Hydroxyméthylpyridin-2-ylamino)pyridin-4-yl]-2-(tétrahydropyran-4-yloxy)benzonitrile |
| « A19 » | 5-{4-[3-Cyano-4-(tétrahydropyran-4-yloxy)phényl]pyridin-2-yl-amino}benzofuran-2-carboxamide |
| « A20 » | 5-{2-[1-(2,2-Difluoroéthyl)-1H-pyrazol-4-ylamino]pyridin-4-yl}-2-(tétrahydropyran-4-yloxy)benzonitrile |
| « A21 » | 5-{2-[1-(2-Pipéridin-4-yléthyl)-1H-pyrazol-4-ylamino]pyridin-4-yl}-2-(tétrahydropyran-4-yloxy)benzonitrile |
| « A22 » | 5-{2-[1-(2-Morpholin-4-yléthyl)-1H-pyrazol-4-ylamino]pyridin-4-yl}-2-(tétrahydropyran-4-yloxy)benzonitrile |
| « A23 » | 5-{2-[1-(3-Méthoxypropyl)-1H-pyrazol-4-ylamino]pyridin-4-yl}-2-(tétrahydropyran-4-yloxy)benzonitrile |
| « A24 » | 5-{2-[1-(2-Cyanocyclopropylméthyl)-1H-pyrazol-4-ylamino]pyridin-4-yl}-2-(tétrahydropyran-4-yloxy)benzonitrile |
| « A25 » | 5-[2-(1-Azétidin-3-yl-1H-pyrazol-4-ylamino)pyridin-4-yl]-2-(tétrahydropyran-4-yloxy)benzonitrile |
| « A26 » | 5-{2-[1-((1S,2S)-2-Hydroxyméthylcyclopropylméthyl)-1H-pyrazol-4-ylamino]pyridin-4-yl}-2-(tétrahydropyran-4-yloxy)-benzonitrile |
| « A27 » | 5-{2-[1-(Tétrahydrofuran-3-ylméthyl)-1H-pyrazol-4-ylamino]-pyridin-4-yl}-2-(tétrahydropyran-4-yloxy)benzonitrile |
| « A28 » | 5-[2-(1-Pyrrolidin-3-yl-1H-pyrazol-4-ylamino)pyridin-4-yl]-2-(tétrahydropyran-4-yloxy)benzonitrile |
| « A29 » | 5-[2-(1-{2-[1-(2-Hydroxyacétyl)pipéridin-4-yl]éthyl}-1H-pyrazol-4-ylamino)pyridin-4-yl]-2-(tétrahydropyran-4-yloxy)benzonitrile |
| « A30 » | 5-[2-(1-{2-[1-(2-Aminoacétyl)pipéridin-4-yl]éthyl}-1H-pyrazol-4-ylamino)pyridin-4-yl]-2-(tétrahydropyran-4-yloxy)benzonitrile |
| « A31 » | 5-[2-(3-tertio-Butylisoxazol-5-ylamino)pyridin-4-yl]-2-(tétrahydropyran-4-yloxy)benzonitrile |
| « A32 » | 2-(Tétrahydropyran-4-yloxy)-5-{2-[1-(3-trifluorométhylphényl)-1H-pyrazol-4-ylamino]pyrimidin-4-yl}benzonitrile |
| « A33 » | 5-[2-(1-Méthyl-1H-pyrazol-3-ylamino)pyrimidin-4-yl]-2-(tétrahydropyran-4-yloxy)benzonitrile |
| « A34 » | 5-[2-(1H-Pyrazol-4-ylamino)pyrimidin-4-yl]-2-(tétrahydropyran-4-yloxy)benzonitrile |
| « A35 » | 5-{2-[1-(2-Méthoxyéthyl)-1H-pyrazol-4-ylamino]pyrimidin-4-yl}-2-(tétrahydropyran-4-yloxy)benzonitrile |
| « A36 » | 2-(Tétrahydropyran-4-yloxy)-5-[2-(5,6,7,8-tétrahydro-pyrido[4,3-d]pyrimidin-2-ylamino)pyridin-4-yl]benzonitrile |
| « A37 » | 6-{4-[3-Cyano-4-(tétrahydropyran-4-yloxy)phényl]pyridin-2-yl-amino}nicotinamide |
| « A38 » | 5-{2-[1-(2-Pyrazol-1-yléthyl)-1H-pyrazol-4-ylamino]pyridin-4-yl}-2-(tétrahydropyran-4-yloxy)benzonitrile |
| « A39 » | 5-{2-[1-(2-Morpholin-4-yléthyl)-1H-pyrazol-4-ylamino]pyrimidin-4-yl}-2-(tétrahydropyran-4-yloxy)benzonitrile |
| « A40 » | 5-[2-(1-Pyrrolidin-3-yl-1H-pyrazol-4-ylamino)pyrimidin-4-yl]-2-(tétrahydropyran-4-yloxy)benzonitrile |
| « A41 » | 5-{2-[1-(Tétrahydrofuran-3-ylméthyl)-1H-pyrazol-4-ylamino]-pyrimidin-4-yl}-2-(tétrahydropyran-4-yloxy)benzonitrile |
| « A42 » | 5-{4-[3-Cyano-4-(tétrahydropyran-4-yloxy)phényl]pyrimidin-2-yl-amino}benzofuran-2-carboxamide |
| « A43 » | 5-{2-[1-(2-Pyrazol-1-yléthyl)-1H-pyrazol-4-ylamino]pyrimidin-4-yl}-2-(tétrahydropyran-4-yloxy)benzonitrile |
| « A44 » | 5-{2-[1-(2,2-Difluoroéthyl)-1H-pyrazol-4-ylamino]pyrimidin-4-yl}-2-(tétrahydropyran-4-yloxy)benzonitrile |
| « A45 » | 5-{2-[1-(2-Pipéridin-4-yléthyl)-1H-pyrazol-4-ylamino]pyrimidin-4-yl}-2-(tétrahydropyran-4-yloxy)benzonitrile |
| « A46 » | 5-{2-[1-(3-Méthoxypropyl)-1H-pyrazol-4-ylamino]pyrimidin-4-yl}-2-(tétrahydropyran-4-yloxy)benzonitrile |
| « A47 » | 5-{2-[5-(3,6-Dihydro-2H-pyran-4-yl)pyridin-2-ylamino]pyridin-4-yl}-2-(tétrahydropyran-4-yloxy)benzonitrile |
| « A48 » | 5-[2-(1',2',3',6'-Tétrahydro-[3,4']bipyridinyl-6-ylamino)pyridin-4-yl]-2-(tétrahydropyran-4-yloxy)benzonitrile |
| « A53 » | 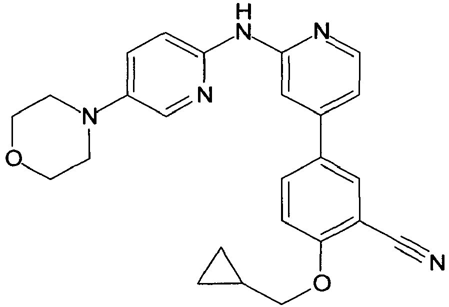 |
| « A58 » | 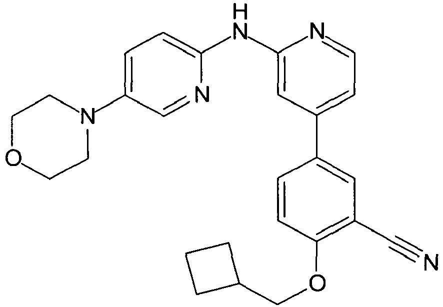 |
| « A59 » | 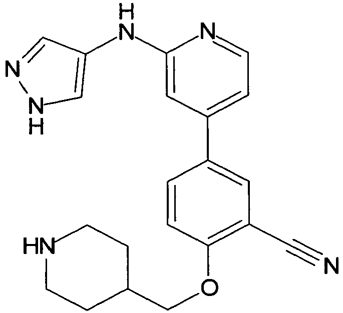 |
| « A60 » | 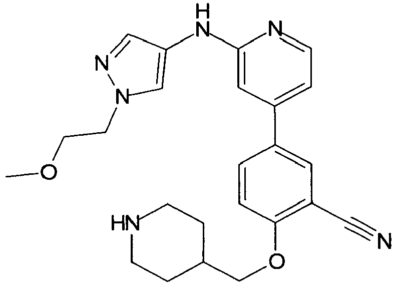 |
| « A61 » | 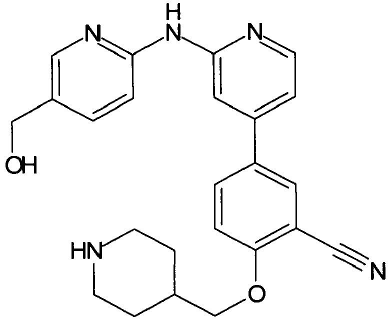 |
| « A62 » | 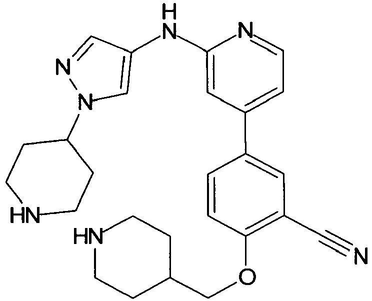 |
| « A63 » | 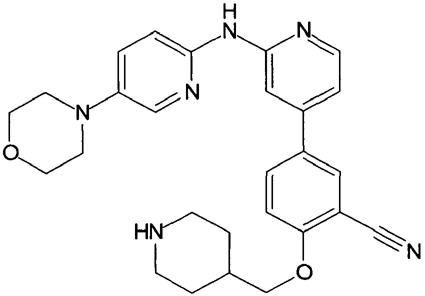 |
ainsi que les sels, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, tautomères et stéréoisomères pharmaceutiquement utilisables de celui-ci, y compris des mélanges de ceux-ci en toutes proportions, et éventuellement des excipients et/ou des adjuvants.

3. Composés selon la revendication 1 et sels, tautomères et stéréoisomères pharmaceutiquement utilisables de ceux-ci, y compris des mélanges de ceux-ci en toutes proportions, pour une utilisation dans le traitement du cancer, du choc septique, du glaucome primitif à angle ouvert (POAG), de l'hyperplasie, de l'athérosclérose, de la rétinopathie, de l'arthrose, de l'endométriose, des inflammations chroniques, des maladies neurodégénératives, de la polyarthrite rhumatoïde (« RA »), du lupus érythémateux disséminé (« SLE »), du syndrome de Sjögren, du syndrome d'Aicardi-Goutières, du lupus engelure, de la vasculopa-thie rétinienne, de la leucodystrophie cérébrale (« RVCL »), de la sclérodermie généralisée, de la myosite, du psoriasis, de la bronchopneumopathie chronique obstructive (BPCO), de l'affection abdominale inflammatoire (« IBD »), de l'obésité, de l'insulinorésistance, du diabète de type 2 (DNID) et/ou du syndrome métabolique.

4. Composés selon la revendication 1 et/ou des sels, tautomères et stéréoisomères physiologiquement acceptables de ceux-ci pour une utilisation dans le traitement de tumeurs, où une quantité thérapeutiquement efficace d'un composé selon la revendication 1 est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

5. Composés selon la revendication 1 et/ou des sels, tautomères et stéréoisomères physiologiquement acceptables de ceux-ci pour une utilisation dans le traitement de tumeurs, où une quantité thérapeutiquement efficace d'un composé selon la revendication 1 est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.


### SEQUENCE LISTING

<110> Merck Patent GmbH
<120> Benzonitrilderivate
<130> P 11/149-ve/ms
<150> DE 102011112978.6
   <151> 2011-09-09
<160> 2
<170> PatentIn version 3.5
<210> 1
   <211> 23
   <212> PRT
   <213> Artificial Sequence
<220>
   <223> Biotin-C6-C6
<220>
   <221> MISC_FEATURE
   <222> (1)..(1)
<400> 1
   Gly Leu Lys Lys Glu Arg Leu Leu Asp Asp Arg His Asp Ser Gly Leu
   1               5                   10                  15
   Asp Ser Met Lys Asp Glu Glu
               20
<210> 2
   <211> 23
   <212> PRT
   <213> Artificial Sequence
<220>
   <223> Biotin-Ah-Ah
<220>
   <221> MISC_FEATURE
   <222> (1)..(1)
<400> 2
   Ala Lys Pro Lys Gly Asn Lys Asp Tyr His Leu Gln Thr Cys Cys Gly
   1               5                   10                  15
   Ser Leu Ala Tyr Arg Arg Arg
               20